(19) Europäisches Patentamt
European Patent Office
Office européen des brevets

(11) EP 1 236 399 A1

(12) # EUROPEAN PATENT APPLICATION
published in accordance with Art. 158(3) EPC

(43) Date of publication:
**04.09.2002 Bulletin 2002/36**

(21) Application number: **00980020.2**

(22) Date of filing: **08.12.2000**

(51) Int Cl.[7]: **A01N 59/08**, A01N 25/30, A01N 25/02, A01N 33/24, A61L 2/18, A23L 3/358, D06M 11/11

(86) International application number:
**PCT/JP00/08717**

(87) International publication number:
**WO 01/041572 (14.06.2001 Gazette 2001/24)**

(84) Designated Contracting States:
**AT BE CH CY DE DK ES FI FR GB GR IE IT LI LU MC NL PT SE TR**

(30) Priority:
**10.12.1999 JP 35221899**
**18.02.2000 JP 2000041093**
**31.03.2000 JP 2000098960**
**31.03.2000 JP 2000098961**
**31.03.2000 JP 2000098962**
**31.03.2000 JP 2000098963**
**31.03.2000 JP 2000098964**
**28.04.2000 JP 2000130278**
**22.06.2000 JP 2000187442**

(71) Applicant: **Kao Corporation**
**Tokyo 103-8210 (JP)**

(72) Inventors:
- **YOSHIKAWA, Kiyoaki**
**Kao Corporation, Research Lab.**
**Wakayama 640-8580 (JP)**
- **YAMAZAKI, Yoshihiro**
**Kao Corporation, Research Lab.**
**Wakayama 640-8580 (JP)**
- **OKANO, Tetsuya**
**Kao Corporation, Research Lab.**
**Wakayama 640-8580 (JP)**
- **ITO, Sumitoshi Kao Corporation, Research Lab.**
**Wakayama 640-8580 (JP)**
- **TAMURA, Shigeru**
**Kao Corporation, Research Lab.**
**Wakayama 640-8580 (JP)**
- **AZUMA, Toshikazu**
**Kao Corporation, Research Lab.**
**deceased (JP)**
- **ITOI, Takashi Kao Corporation, Research Lab.**
**Wakayama 640-8580 (JP)**
- **MATSUO, Noboru**
**Kao Corporation, Research Lab.**
**Wakayama 640-8580 (JP)**
- **NIKI, Masao Kao Corporation, Research Lab.**
**Wakayama 640-8580 (JP)**
- **NAKANE, Shoji Kao Corporation, Research Lab.**
**Wakayama 640-8580 (JP)**
- **SAITO, Shinya Kao Corporation, Research Lab.**
**Wakayama 640-8580 (JP)**

(74) Representative: **HOFFMANN - EITLE**
**Patent- und Rechtsanwälte**
**Arabellastrasse 4**
**81925 München (DE)**

(54) **METHODS OF STERILIZATION**

(57) The present invention provides a method for carrying out a germicidal process on microbes, which uses hypochlorous acid or the like. More specifically, the present invention provides a sterilizing method in which an aqueous solution comprising (A) hypochlorous acid and/or a salt thereof, (B) a surfactant and (C) a pH adjusting agent is made in contact with microbes.

EP 1 236 399 A1

## Description

Technical field

The present invention relates to a method for sterilizing microbes in which hypochlorous acid or the like is used.

Prior Art

Conventionally, chlorine-based germicides such as sodium hypochlorite, calcium hypochlorite and sodium dichloroisocyanurate have been generally used in various environments as germicides and disinfectants. Among them, hypochlorites such as sodium hypochlorite are widely used, since these are advantageous in costs and effects, and there have been many proposals so as to further improve the effects thereof with respect to germicidal action and sterilizing action to microbes that are requested in various fields such as medical and food industries.
For example, JP-A No. 57-61099 has disclosed a liquid germicidal bleaching agent that comprises a hypochlorite, an alkali substance and a specific cationic surfactant of a quaternary ammonium salt type in respectively specific weight ratios.
JP-A No. 7-233396 has disclosed a germicidal washing agent for use in medical equipment such as artificial dialysis devices, which comprises sodium hypochlorite, an anionic surfactant, an alkali agent, an anion surfactant and a chelating agent.
However, although conventional hypochlorite germicides are effective to general bacteria and mold (mycelia) to a certain degree, these fail to provide sufficient effects to virus having higher resistance to medicine, spores formed by rod-shaped bacteria and mold spores in the case of an easy operation.
Moreover, JP-A No. 11-148098 has disclosed a solid-state germicidal washing agent in which chlorine-based germicides such as an alkaline earth metal of hypochlorous acid like high test hypochlorite (calcium hypochlorite), solid state acid and a surfactant are used; however, this has not disclosed anything about germicidal processes in a higher degree, and alkaline earth metal such as calcium causes scales and scum, resulting in degradation in the germicidal effects.
Furthermore, JP-A No. 7-328638 has disclosed a method in which a surfactant for reducing surface tension is added to electrolytic acidic water so as to increase the adhering property to the outer surface of a germicide-subject substance; however, although this method is superior in the germicidal effects, it generates chlorine gas, causing a problem with safety.
Here, JP-A No. 59-93799 has disclosed a method in which amine oxide is blended in a liquid washing agent comprising a hypochlorite and an alkali substance.
Further, JP-A No. 59-98200 has disclosed a method in which amine oxide is used as a thickener for a bleaching agent comprising alkali metal hypochlorite; however, these have not described anything about germicidal effects, that is, in particular, germicidal effects to spores and viruses having high resistance.

Disclosure of the Invention

The objective of the present invention is to obtain a sterilizing method which provide a high germicidal effect through a simple process, and is superior in safety and operability.
The present invention is a method for sterilizing microbes, comprising bringing an aqueous solution comprising (A) hypochlorous acid and/or a salt thereof, (B) a surfactant and (C) a pH adjusting agent in contact with microbes. In other words, this is a method of sterilizing mirobes, comprising applying an effective amount for germicide of an aqueous solution comprising (A) hypochlorous acid and/or its salt, (B) a surfactant and (C) a pH adjusting agent to a place from which microbes are eliminated so that the germicidal operation is carried out.
More preferably, the aqueous solution is set to have a pH value (25°C) of 3 to 8, and an available chlorine concentration of 5 to 5000 ppm. The pH value (25°C) is preferably set to 5 to 8, more preferably, 5 to 7.5, most preferably, not less than 5 to less than 7, by far the most preferably, not less than 6 to less than 7.
A preferable pH adjusting agent (C) is an organic acid or a salt thereof, and, in particular, the organic acid or a salt thereof is a saturated dibasic acid or a salt thereof. The saturated organic acid or a salt thereof is preferably contained in a pH value (25°C) of not less than 5 to less than 7, more preferably, not less than 6 to less than 7.
A preferable surfactant (B) is not less than one kind selected from the group consisting of an amphoteric surfactant, a cationic surfactant and a nonionic surfactant. More preferably, it is not less than one kind selected from the group consisting of surfactants of polyhydric derivative type that are amphoteric surfactants, cationic surfactants or nonionic surfactants. With respect to the amphoteric surfactants, amine oxide is preferably used.
Moreover, sodium hypochlorite (A), lauryldimethylamine oxide (B) and succinic acid (C), etc. are preferably used.

The above-mentioned components (A), (B) and (C) are included in any one of the following preferred embodiments (R) to (X).

Moreover, the pH adjusting methods, additives and application methods disclosed in any one of the following preferred embodiments (R) to (X) are included in the scope of the present invention.

The method for sterilizing microbes of the present invention specifically include the following preferred examples. The specific applications include a sterilizing process on hard surfaces, a mold eliminating process, a sterilizing process for automatic washers, a sterilizing process for perishable foodstuff, a sterilizing process for textiles and fabrics and a sterilizing process for medical tools. The method is also applied to food processing and a sterilizing process for kitchens. The method is further applied to a sterilizing and washing process for plastic containers of drinking water. The method is also applied to a sterilizing and washing process for recycled bottles.

Preferred Embodiment (R)

In preferred embodiment (R), in the above-mentioned invention, an alkali metal hypochlorite is used as (A) component, a surfactant of amine oxide or a surfactant of polyhydric alcohol derivative type is used as (B) component and an organic acid or a salt thereof is used as (C) component.

With respect to (A) an alkali metal hypochlorite used in the present invention, examples thereof include sodium hypochlorite, potassium hypochlorite and lithium hypochlorite, and among them, sodium hypochlorite is preferably used.

With respect to (B) amine oxide, for example, alkyldimethylamine oxides are used, and in particular, those oxides including an alkyl group having carbon atoms of 8 to 18 are more preferably used.

With respect to an aqueous solution used in preferred embodiment (R) of the present invention, when an alkali metal hypochlorite (A) and an amine oxide (B) are combinedly used, the sterilizing effect is cooperatively improved, and the aqueous solution preferably comprises (A) component and (B) component at a ratio of (A)/(B) = 10/1 to 1/10, more preferably, 5/1 to 1/5, most preferably, 2/1 to 1/2 in the weight ratio.

Here, the weight of (A) component is based upon effective chlorine.

The aqueous solution of the present invention preferably comprises an organic acid or a salt thereof (C). With respect to the organic acid or a salt thereof (C), examples thereof include saturated dibasic acids such as malonic acid, succinic acid, glutaric acid, adipic acid and sebacic acid or salts thereof, and unsaturated dibasic acids such as fumaric acid and maleic acid or salts thereof. Preferably, those are saturated dibasic acids or salts thereof, more preferably, saturated dibasic acids or salts thereof having carbon atoms of 3 to 10, most preferably, succinic acid or a salt thereof. The organic acid or a salt thereof (C) is preferably used with an alkali metal hypochlorite (A) at a weight ratio of (C)/(A) = 5/1 to 1/10, more preferably, 2/1 to 1/5, most preferably, 1/1 to 1/5.

The aqueous solution of the present invention may comprise a hydroxide of an alkali metal and/or a hydroxide of an alkaline earth metal (D). With respect to (D), preferable examples thereof include sodium hydroxide, potassium hydroxide and calcium hydroxide, and sodium hydroxide and potassium hydroxide are preferably used.

The aqueous solution of the present invention may comprise an alkali metal salt of inorganic acid and/or an alkaline earth metal salt (E) of inorganic acid. With respect to (E) component, sodium sulfate, sodium nitrate, sodium chloride, sodium carbonate, sodium hydrogencarbonate, magnesium sulfate, magnesium nitrate, magnesium chloride, magnesium carbonate, sodium phosphate, sodium polyphosphate and potassium phosphate; and, sodium sulfate, magnesium sulfate, sodium phosphate, sodium polyphosphate and potassium phosphate are preferably used.

The aqueous solution used in the present invention is preferably set to have a pH value (25°C) of 3 to 8, preferably, 5 to 8, more preferably, 5 to 7.5, most preferably, from not less than 5 to less than 7, by far the most preferably, from not less than 6 to less than 7, and the adjustment of pH is carried out by using the above-mentioned organic acid or salt (C) thereof and inorganic acid. Moreover, the aqueous solution is preferably set to have an available chlorine concentration of 5 to 5000 ppm, more preferably, 50 to 200 ppm.

In the state of a blended solution, the aqueous solution of the present invention is preferably set to have 5 ppm to 12 weight % of (A) component, more preferably, 10 to 60000 ppm, 0.5 ppm to 35 weight % of (B) component, more preferably, 1 ppm to 10 weight %, and 0.5 ppm to 60 weight % of (C) component, more preferably, 1 ppm to 10 weight %. Normally, an aqueous solution, prepared by further diluting this aqueous solution, is made in contact with microbes; and this diluted aqueous solution is preferably set to comprise 5 to 5000 ppm, more preferably 10 to 5000 ppm, the most preferably 50 to 200 ppm, of the component (A); 0.5 to 50000 ppm, more preferably 5 to 2000 ppm, the most preferably 50 to 200 ppm, of the component (B); 0.5 to 25000 ppm, more preferably 5 to 1000 ppm, the most preferably 25 to 500 ppm, in particular 25 to 150 ppm, of the component (C).

In the sterilizing method of the present invention, an aqueous solution comprising the above-mentioned (A) component and (B) component and further (C) to (E) components is made in contact with microbes. The microbes refer to general bacteria, mold fungi, virus, mold spores, bacteria spores, etc.

The contact method of the aqueous solution is not particularly limited, and methods such as spraying, atomizing, immersion and filling methods may be used, or a subject matter may be wiped by an appropriate bearing member which is impregnated with the aqueous solution. The contact time of the aqueous solution also is not particularly limited, and even such a short contact time of less than 30 seconds, and in particular, less than 10 seconds, may provide sufficient effects depending on microbes. Although not particularly limited, the temperature of the aqueous solution to be made in contact is preferably set in the range of 10 to 70°C, more preferably, 20 to 60°C.

The sterilizing method of the present invention, which has wider sterilizing spectra, is highly effective not only to bacteria (mold), but also to virus and spores; therefore, it is useful as a sterilizing method in wide fields. For example, it is applied to sterilizing processes of walls, floors, windows, etc., of hospitals, nursing homes, food processing factories, cleaning facilities, kitchens, etc. or tools, equipment and containers of products (for example, drinking water) that are used therein.

The sterilizing method of the present invention exhibits superior effects to various bacteria, in particular, bacteria having high resistance such as spores and virus, and is also superior in safety and operability.

Moreover, in the present invention, since an aqueous solution comprising an alkali metal hypochlorite is applied, it is possible to easily carry out a concentration-adjusting process and a diluting process, etc. using an automatic supplying apparatus, and also to provide a superior handling property in comparison with powder bactericides such as bleaching powder (calcium hypochlorite).

Preferred Embodiment (S)

In preferred embodiment (S), in the above-mentioned invention, not less than one kind selected from the group consisting of a hypochlorite and hypochlorous acid is used as (A) component, and not less than one kind selected from the group consisting of an amphoteric surfactant and a cationic surfactant is used as (B) component. Alternatively, at least one kind of nonionic surfactant may be used.

With respect to the hypochlorite of (A) component, examples thereof include alkali metal hypochlorites such as potassium hypochlorite and sodium hypochlorite, and alkaline earth metal hypochlorites such as calcium hypochlorite and magnesium hypochlorite, and among them, alkali metal hypochlorites are preferably used, and sodium hypochlorite is more preferably used. With respect to (A) component, the composition is preferably set to have an available chlorine concentration of 1 to 500 ppm, more preferably, 10 to 300 ppm, most preferably, 30 to 100 ppm.

With respect to the amphoteric surfactant of (B) component, examples thereof include amine oxides such as alkyldimethylamine oxides, and betaines such as alkyldimethyl aminofatty acid betaines and alkylcarboxymethylhydroxyethylimidazolium betaines. Among them, alkyldimethylamine oxides having an alkyl group having carbon atoms of 8 to 18 are preferably used. Moreover, with respect to cationic surfactants of (B) component, the primary amine salts, the secondary amine salts, the tertiary amine salts and the quaternary ammonium salts are preferably listed, and among them, the quaternary ammonium salts are more preferably used. With respect to the quaternary ammonium salts, for example, such a compound that has an alkyl or alkenyl group having the total number of carbon atoms of 8 to 28 as at least one of the four substitutes, with the rest of groups being groups selected from a benzyl group, an alkyl group having carbon atoms of 1 to 5 and a hydroxy alkyl group having carbon atoms of 1 to 5 is listed. The alkyl group or alkenyl group having the total number of carbon atoms of 8 to 28 may be substituted by an alkoxyl group, alkenyl oxy group, alkenoyl amino group, alkenoyl amino group, alkanoyl oxy group or an alkenoyl oxy group, within the range of this range of number of carbon atoms.

The composition of the present invention is preferably set to have (B) component in a range of 1 to 5000 ppm, more preferably, 5 to 3000 ppm, most preferably, 10 to 1000 ppm.

Moreover, with respect to the composition of preferred embodiment (S) of the present invention, the weight ratio of (A) component and (B) component is the same as (A)/(B) described in preferred embodiment (R).

With respect to pH adjusting agent (C) , examples thereof include a hydroxide of alkali metal, a hydroxide of alkaline earth metal, an inorganic acid or a salt thereof, an organic acid or a salt thereof, etc. With respect to the hydroxide of alkali metal or hydroxide of alkaline earth metal, those compounds described in the above-mentioned components (D), such as sodium hydroxide, potassium hydroxide and calcium hydroxide, may be listed. With respect to the inorganic acid or a salt thereof, examples thereof include hydrochloric acid, sulfuric acid, sodium sulfate, sodium nitrate, sodium chloride, sodium carbonate, potassium hydrogencarbonate, sodium hydrogencarbonate, potassium hydrogencarbonate, magnesium sulfate, magnesium nitrate, magnesium chloride, magnesium carbonate, sodium triphosphate, potassium triphosphate, disodium hydrogenphosphate, dipotassium hydrogenphosphate, sodium dihydrogenphosphate, potassium dihydrogenphosphate, and sodium polyphosphate.

With respect to the organic acid and a salt thereof, those described in preferred embodiment (R) are also used.

The composition (S) used in the present invention is preferably set to have a pH value (20°C) of 3 to 8, preferably, 5 to 8, more preferably, 5 to 7.5, most preferably, from not less than 5 to less than 7, by far the most preferably, from not less than 6 to less than 7. The amount of application of component (C) is preferably set so as to set the pH value in the above-mentioned range.

The composition (S) of the present preferred embodiment of the present invention may comprise an anionic surfactant. With respect to anionic surfactants, examples thereof include: salts of higher fatty acid, higher alcohol sulfates, higher alcohol sulfonates, salts of sulfated fatty acid, salts of sulfonated fatty acid, phosphates, sulfates of fatty acid ester, sulfonates of fatty acid ester, sulfates of higher alcohol ether, sulfonates of higher alcohol ether, acetates of ether-substituted higher alcohol, condensation products of fatty acid and amino acid, alkylolated sulfates of fatty acid amide, alkylated sulfonates of fatty acid amide, sulfosuccinates, alkyl benzenesulfonates, alkyl phenolsulfonates, alkyl naphthalenesulfonates, alkyl benzoimidazolesulfonates, amide ether carboxylates or salts thereof, ether carboxylates or salts thereof, N-acyl-N-methyltaurine or salts thereof, amide ether sulfuric acid or salts thereof, N-acyl glutamic acid or salts thereof, N-amide ethyl-N-hydroxyethyl acetic acid or salts thereof, acyloxyethane sulfonic acid or salts thereof, N-acel-β-alanine or salts thereof, N-acey-N-carboxylethyl taurine or salts thereof, N-acyl-N-carboxyethyl glycine or salts thereof, and alkyl or alkenyl aminocarbonylmethyl sulfuric acid or salts thereof. The amount of blend of the anionic surfactant is set to 1 to 1000 ppm, more preferably, 5 to 500 ppm, most preferably, 10 to 200 ppm, in the composition.

In addition to the above-mentioned components (A) to (C), the germicide composition used for an automatic washing machine of the present invention may comprise conventionally known components including inorganic builders, such as tripolyphosphates, pyrophosphates, carbonates, percarbonates, silicates and sulfates, organic builders, such as ethylene diamine tetraacetates, aminotrimethylene phosphonates, 1-hydroxy-1,1-diphosphonates, ethylenediaminetetramethylene phosphonates, diethylene triaminepentamethylene phosphonates, citrates, gluconates, polyacrylates, copolymer of acrylic acid-maleic acid and carboxymethyl celluloses, and low foaming nonionic surfactants and enzymes.

The composition of the present invention is preferably applied to automatic washers used for washing dishes, etc. Here, the automatic washers refer to the entire apparatus which can wash hard surfaces such as dishes like cups, transporting-use containers like plastic containers, continuously or in a batch system, and the size and method thereof are not particularly limited. The sterilizing process using this is more effective when carried out after removing stains, and, for example, in the case of an automatic dish washer of a conveyor belt type, it is preferable to spray the composition of the present invention prior to a final rinsing process after the washing process.

The composition of the present invention is preferably applied to automatic washers that are used for washing dishes, etc., and exerts a high germicidal function even to bacteria having high resistance such as spore forming bacteria, even when used in a normal operation.

Preferred Embodiment (T)

In preferred embodiment (T), in the above-mentioned invention, not less than one kind selected from the group consisting of a hypochlorite and hypochlorous acid is used as (A) component, and not less than one kind selected from the group consisting of an amphoteric surfactant and a cationic surfactant is used as (B) component. Alternatively, at least one kind of nonionic surfactant may be used.

With respect to the hypochlorite of (A) component, the same composition as described in preferred embodiment (S) are used. With respect to (A) component, the composition is preferably set to have an available chlorine concentration of 1 to 2000 ppm, more preferably, 10 to 1000 ppm, most preferably, 50 to 500 ppm.

With respect to the amphoteric surfactant or cationic surfactant of (B) component, the same composition as described in preferred embodiment (S) are used.

The composition of the present invention is preferably set to comprise 1 ppm to 2 weight % of component (B), more preferably, 5 ppm to 1 weight %, most preferably, 10 to 5000 ppm.

With respect to the composition of the present preferred embodiment (S), the weight ratio of (A) component and (B) component is set in the same manner as described as (A)/(B) in preferred embodiment (R).

Examples of pH adjusting agent (C) are the same as those described in preferred embodiment (S).

In the composition of preferred embodiment (T) of the present invention, the pH (20°C) value thereof may be adjusted in the same manner as preferred embodiment (S).

Moreover, the composition of preferred embodiment (T) of the present invention may comprise an anionic surfactant in the same manner as preferred embodiment (S). The examples of the anionic surfactant are the same as those shown in preferred embodiment (S). The amount of blend of the anionic surfactant is preferably set to 1 ppm to 5 weight %, more preferably, 5 ppm to 1 weight %, most preferably, 10 to 5000 ppm, in the composition.

With respect to textiles and fabrics that form subjects to which the composition of the present invention is applied, examples include diapers, wiping towels, sheets and pajamas. The composition of the present invention is effective when applied to these textiles and fabrics after stains have been removed from the textiles and fabrics in a preliminary washing process and a main washing process, and, in particular, is effectively applied to a rinsing process prior to a final rinsing process.

The composition of the present invention makes it possible to provide a germicidal composition for textiles and fabrics, which exerts a high germicidal function even to bacteria having high resistance such as spore forming bacteria, even when used in a normal operation.

Preferred Embodiment (U)

In preferred embodiment (U), in the above-mentioned invention, not less than one kind selected from the group consisting of a hypochlorite and hypochlorous acid is used as (A) component, and not less than one kind selected from the group consisting of an amphoteric surfactant and a cationic surfactant is used as (B) component. Alternatively, at least one kind of nonionic surfactant may be used.
With respect to the hypochlorite of (A) component, the same composition as described in preferred embodiment (S) is used. With respect to (A) component, the composition is preferably set to have an available chlorine concentration of 1 to 1000 ppm, more preferably, 10 to 500 ppm, most preferably, 50 to 200 ppm.
With respect to the amphoteric surfactant or cationic surfactant of (B) component, the same composition as described in preferred embodiment (S) is used. Here, the composition is preferably set to comprise 1 ppm to 1 weight % of component (B), more preferably, 1 ppm to 5000 ppm, most preferably, 5 to 2000 ppm.
With respect to the composition of the present preferred embodiment (U), the weight ratio of (A) component and (B) component is set in the same manner as described as (A) / (B) in preferred embodiment (R).
Examples of pH adjusting agent (C) are the same as those described in preferred embodiment (S). Moreover, the organic acid and a salt thereof are set in the same manner as preferred embodiment (R).
In the composition of preferred embodiment (U) of the present invention, the pH value thereof may be adjusted in the same manner as preferred embodiment (S).
The composition of preferred embodiment (U) of the present invention may comprise an anionic surfactant in the same manner as preferred embodiment (S) . Examples of the anionic surfactant are the same as those described in preferred embodiment (S). The amount of blend of the anionic surfactant is preferably set to 1 ppm to 1 weight %, more preferably, 1 to 5000 ppm, most preferably, 5 to 2000 ppm, in the composition.
The present preferred embodiment (U) is effectively used as germicidal washing composition for perishable foodstuff, and may comprise organic builders, low foaming nonionic surfactants and enzymes, on demand, as described in preferred embodiment (S) in addition to the above-mentioned (A) to (C) components. Therefore, this is also preferably applied to automatic washers. With respect to the germicidal washing process of perishable foodstuff, in the case of much adhesion of organic stains, after these stains have been removed, a germicidal washing process is carried out thereon by using the composition of the present invention effectively.
In accordance with the present preferred embodiment, it is possible to provide a germicidal washing agent composition for perishable foodstuff, which exhibits superior germicidal washing effects with good safety conditions. The composition of the present invention is desirably applicable to automatic washers for perishable foodstuff.

Preferred Embodiment (V)

In preferred embodiment (V), in the above-mentioned invention, not less than one kind selected from the group consisting of a hypochlorite and hypochlorous acid is used as (A) component, and not less than one kind selected from the group consisting of an amphoteric surfactant and a cationic surfactant is used as (B) component. Alternatively, at least one kind of nonionic surfactant may be used.
With respect to the hypochlorite of (A) component, the same composition as described in preferred embodiment (S) is used. With respect to (A) component, the composition is preferably set to have an available chlorine concentration of 1 to 10000 ppm, more preferably, 10 to 5000 ppm, most preferably, 50 to 2000 ppm.
With respect to the amphoteric surfactant or cationic surfactant of (B) component, the same composition as described in preferred embodiment (S) is used. Here, the composition is preferably set to comprise 1 ppm to 10 weight % of component (B), more preferably, 10 ppm to 5 weight %, most preferably, 50 ppm to 2 weight %.
With respect to the composition of the present preferred embodiment (V), the weight ratio of (A) component and (B) component is set in the same manner as described as (A)/(B) in preferred embodiment (R).
Examples of pH adjusting agent (C) are the same as those described in preferred embodiment (S) Moreover, examples of the organic acid and a salt thereof are the same as those described in preferred embodiment (R).
In the composition of preferred embodiment (V) of the present invention, the pH (20°C) value thereof may be adjusted in the same manner as preferred embodiment (S).
Moreover, the composition of preferred embodiment (V) of the present invention may comprise an anionic surfactant in the same manner as preferred embodiment (S). The examples of the anionic surfactant are the same as those shown in preferred embodiment (S). The amount of blend of the anionic surfactant is preferably set to 1 ppm to 50 weight %, more preferably, 10 ppm to 5 weight %, most preferably, 50 ppm to 2 weight %, in the composition.
The present preferred embodiment is useful for virucide composition, and in addition to the above-mentioned components (A) to (C), may comprise, on demand, inorganic builders, such as tripolyphosphates, pyrophosphates, carbonates, percarbonates, silicates and sulfates, organic builders, such as ethylenediamine tetraacetates, aminotrimethylene phosphonates, 1-hydroxy-1,1-diphosphonates, ethylenediaminetetramethylene phosphonates, diethylene triaminepentamethylene phosphonates, citrates, gluconates, polyacrylates, copolymer of acrylic acid-maleic acid and carboxymethyl celluloses, and low foaming nonionic surfactants and enzymes.

The composition of the present invention is preferably applied to automatic washers used for washing dishes, etc. Here, the automatic washers refer to general apparatuses which automatically wash endoscopes and medical devices, and are not limited by their types, etc. On principle, it is preferable to carry out a virus-eliminating process using this composition after a washing process to obtain better effects.

The present invention makes it possible to provide a virucide composition which exerts a high virucidal function with good safety conditions and superior operability.

Preferred Embodiment (W)

In preferred embodiment (W) in the above-mentioned invention, not less than one kind selected from the group consisting of a hypochlorite and hypochlorous acid is used as (A) component, and not less than one kind selected from the group consisting of an amphoteric surfactant and a cationic surfactant is used as (B) component. Alternatively, at least one kind of nonionic surfactant may be used.

With respect to the hypochlorite of (A) component, the same composition as described in preferred embodiment (S) is used. With respect to (A) component, the composition is preferably set to have an available chlorine concentration of 1 to 5000 ppm, more preferably, 10 to 1000 ppm, most preferably, 50 to 500 ppm.

With respect to the amphoteric surfactant or cationic surfactant of (B) component, the same composition as described in preferred embodiment (S) are used. The composition of the present invention is preferably set to comprise 1 ppm to 5 weight % of component (B), more preferably, 5 ppm to 1 weight %, most preferably, 10 to 5000 ppm.

With respect to the composition of the present preferred embodiment (W), the weight ratio of (A) component and (B) component is set in the same manner as described as (A)/(B) in preferred embodiment (R).

Examples of pH adjusting agent (C) are the same as those described in preferred embodiment (S) Moreover, examples of the organic acid and a salt thereof are the same as those described in preferred embodiment (R).

The pH of the composition of the present preferred embodiment (W) is adjusted in the same manner as preferred embodiment (S).

The composition of the present preferred embodiment provides a superior mold-eliminating effect even in a short-time treatment with a low concentration, in comparison with conventional mold-eliminating agents. This effect is obtained by a cooperative effect between a high oxidizing property of the hypochlorite that decomposes stain components and high permeability of the amphoteric surfactant or cationic surfactant. Moreover, in general, the oxidizing and reducing electric potential of a hypochlorite is higher in the neutral range than in the alkali range; therefore, the composition of the present invention makes it possible to maintain a high detergency even in the application in the neutral range, which is a safer application.

Moreover, the composition of the present invention, which achieves a stable blend among the hypochlorite and/or a salt thereof and other components, may also comprise an anionic surfactant in order to enhance the washing effect. The examples of the anionic surfactant are the same as those shown in preferred embodiment (S). The amount of blend of the anionic surfactant is preferably set to 1 ppm to 5 weight %, more preferably, 10 ppm to 0.5 weight %, most preferably, 50 to 500 ppm, in the composition.

The present invention makes it possible to provide a mold-eliminating composition that exerts superior mold-eliminating effects even when used in the same manner as generally-used mold-eliminating agents.

Preferred Embodiment (X)

In preferred embodiment (X) in the above-mentioned invention, not less than one kind selected from the group consisting of a hypochlorite and hypochlorous acid is used as (A) component, and not less than one kind selected from the group consisting of an amphoteric surfactant and a cationic surfactant is used as (B) component. Alternatively, at least one kind of nonionic surfactants may be used.

With respect to the hypochlorite of (A) component, the same composition as described in preferred embodiment (S) is used. With respect to (A) component, the composition is preferably set to have an available chlorine concentration of 1 to 5000 ppm, more preferably, 10 to 1000 ppm, most preferably, 50 to 500 ppm.

With respect to the surfactant of (B) component, the same composition as described in preferred embodiment (S) are used, and examples of the amphoteric surfactant and examples of the cationic surfactant are the same as those described in preferred embodiment (S). The composition of the present invention is preferably set to comprise 1 ppm to 5 weight % of component (B), more preferably, 5 ppm to 1 weight %, most preferably, 10 to 5000 ppm.

With respect to the composition of the present preferred embodiment (X), the weight ratio of (A) component and (B) component is set in the same manner as described as (A)/(B) in preferred embodiment (R).

Examples of pH adjusting agent (C) are the same as those described in preferred embodiment (S) Moreover, examples of the organic acid and a salt thereof are the same as those described in preferred embodiment (R).

The pH of the composition of the present preferred embodiment (X) is adjusted in the same manner as preferred embodiment (S).

The composition of the present preferred embodiment (X) may also comprise an anionic surfactant in order to improve the permeability to stains. Examples of the anionic surfactant are the same as those described in preferred embodiment (S). The amount of blend of the anionic surfactant is preferably set to 1 ppm to 5 weight %, more preferably, 10 ppm to 0.5 weight %, most preferably, 50 to 500 ppm, in the composition.

The composition of the present preferred embodiment is desirably applicable to a system using an automatic spraying device or a spray gun. Moreover, the composition makes it possible to carry out a foam washing and sterilizing operation by adding a foam-increasing agent thereto.

The present invention makes it possible to provide a mold-eliminating composition for hard surfaces, which exerts a superior detergency on hard surfaces, and also provides superior germicidal effects even on spores and mold even in a treatment at a lower temperature in a shorter time in comparison with normal treatments.

Preferred Embodiment (Z)

In the present invention a nonionic surfactant may be used as (B) component. Those disclosed in the above-mentioned preferred embodiments (R) to (X) may be respectively used. With respect to the nonionic surfactant, those except for polyoxyalkylenealkyl ether and polyoxyalkylenealkylphenyl ether are preferably used. Specific examples include polyhydric alcohol derivative type surfactants, such as sorbitan fatty acid esters, polyoxyalkylene sorbitan fatty acid esters, glycerin fatty acid esters, polyoxyalkylene glycerin fatty acid esters, polyglycerin fatty acid esters, polyoxyalkylenepolyglycerin fatty acid esters, saccharose fatty acid esters, alkyleneglycol fatty acid esters, polyoxyalkyleneglycol fatty acid esters, alkyl(poly)glycoside and polyoxyalkylenealkyl(poly)glycoside, polyoxyalkylene fatty acid esters, resinates and polyoxyalkylene resinates. Among them, polyhydric alcohol derivative type surfactants are preferably used. Among polyhydric alcohol derivative type surfactants, glycerin fatty acid esters, polyglycerin fatty acid ester, propyleneglycol fatty acid ester, polypropyleneglycol fatty acid ester, saccharose fatty acid ester, sorbitan fatty acid ester and alkylpolyglycoside are preferably used, and in particular, polyglycerin fatty acid ester, saccharose fatty acid ester and alkylpolyglcoside are more preferably used.

Examples

All the deposited strains of microorganism, applied to the following examples, can be supplied from the Institute For Fermentation, OSAKA, IFO, located in 17-85, 2-Chome, Jyuso Honmachi, Yodogawa-ku, Osaka City, Osaka Prefecture, Japan. These are described in Microorganisms $10^{th}$ List of Cultures published in 1996.

Examples R1 to R11 and Comparative Examples R1 to R3

By using an aqueous solution composed of components shown in Table R1, the following tests were carried out. Table R1 shows the results of the tests. Here, in Table R1, the available chlorine concentration was measured based upon "Iodometry" in JIS K-0101.

Here, aqueous solution (1) in examples R1 to R11 was prepared by mixing the following solution and pH adjusting agent in the same amount: a solution, which had been obtained by mixing an aqueous solution of sodium hypochlorite (available chlorine concentration 60000 ppm) with a predetermined amount of a surfactant, was diluted by ion exchanged water to two times the final blended concentration to prepare the solution, and a pH adjusting agent such as succinic acid, which had been diluted by adding ion exchanged water to two times the final blended concentration.

[R1] Sporicide test

(1) *Bacillus cereus,* IFO13494, and (2) *Bacillus subtilis,* ATCC6051, which are spore-forming bacteria, were used, and these were subjected to a heating treatment by using a conventional method; thus, the resulting spores were used for the test. A loop of bacteria, which had been preliminarily cultivated in an SCD agar (made by Nihonseiyaku Co., Ltd.) was suspended in 1 ml of sterilized water, and subjected to a heating treatment at 65°C for 30 minutes, and this was then washed while being subjected twice to centrifugal separation; thus, the resulting bacteria were used in the test.

Then, 0.1 ml of this sample spore-containing solution (approximately, $10^7$ to $10^8$ cell/ml) was taken, and made in contact with 10 ml of a test-use aqueous solution (temperature: 25 °C) for 10 seconds which had been prepared by further diluting aqueous solution (1) composed of components listed in table R1 with sterilized ion exchanged water at a rate shown in Table R1, and 50µl of this was sampled and inoculated onto a micro-plate (made by CORNING Co., Ltd., 96-Cell Wells) containing 0.2 ml of post-cultivation-use SCDLP medium (containing 3.3 % of sodium thiosulfate) . This was cultivated at 30°C for 48 hours, and the growth of the bacteria was visually observed; thus, it was determined whether or not the bacteria had grown up on the micro-plate. When no growth of bacteria was observed, this case was evaluated as "○", and when any growth was observed, this case was evaluated as "×".

[R2] Moldicide test

Mold (fungi: Aspergillus niger IFO6341) was cultivated as test-subject bacteria in PDA culture medium at 25°C for 7 days. The resulting mycobiont was uniformed by using a glass bead sterilizing method, and foreign matters were removed therefrom by sterilizing gauze, thereby obtaining a solution containing bacteria. Then, 0.1 ml of this solution containing bacteria (approximately, $10^7$ to $10^8$ cell/ml) was taken, and made in contact with 10 ml of an aqueous solution (temperature: 25 °C) for 10 seconds which had been diluted by ion exchanged water obtained by further sterilizing an aqueous solution comprising components listed in Table R1 at a rate shown in Table R1, and 0.1 ml of this was sampled, and inoculated onto post-cultivation-use PDA medium (containing 3.3 % of sodium thiosulfate) . This was cultivated at 25°C for 7 days, and the growth of the bacteria was visually observed, and evaluated in the same manner as described above.

Table R 1

| | | | Example | | | | | | | | | | | Comparative example | | |
|---|---|---|---|---|---|---|---|---|---|---|---|---|---|---|---|---|
| | | | R1 | R2 | R3 | R4 | R5 | R6 | R7 | R8 | R9 | R10 | R11 | R1 | R2 | R3 |
| Blended components (weight %)of aqueous solution(1) | (A) | Sodium hypochlorite[Available chlorine concentration in aqueous solution (1) is shown in parentheses] | 1.05 (1) | 1.05 (1) | 0.525 (0.5) | 0.525 (0.5) | 1.05 (1) | 1.05 (1) | 1.05 (1) | 1.05 (1) | 1.05 (1) | 1.05 (1) | 1.05 (1) | 1.05 (1) | 1.05 (1) | 1.05 (1) |
| | (B) | Lauryldimethylamine oxide* | 1 | 0.5 | | 0.5 | 1 | 0.5 | | | | | | | | |
| | | Myristyldimethylamine oxide | | | 0.5 | | | | | | | | | | | |
| | | Polyglycerin fatty acid ester** | | | | | | | 1 | 0.5 | | | | | | |
| | | Sucrose fatty acid ester*** | | | | | | | | | 0.5 | | | | | |
| | | Alkylpolyglycoside**** | | | | | | | | | | 1 | 0.5 | | | |
| | (C) | Succinic acid | 0.4 | 0.2 | 0.5 | 0.5 | 0.85 | 1.0 | 0.85 | 1 | 1 | 0.85 | 1 | 0.5 | | 0.85 |
| | Water | | Balance | Balance | Balance | Balance | Balance | Balance | Balance | Balance | Balance | Balance | Balance | Balance | Balance | Balance |
| | Total | | 100 | 100 | 100 | 100 | 100 | 100 | 100 | 100 | 100 | 100 | 100 | 100 | 100 | 100 |
| Test solution | | Dilution ratio of aqueous solution (1) | 20 | 20 | 100 | 50 | 20 | 20 | 20 | 20 | 20 | 20 | 20 | 20 | 20 | 20 |
| | | Available chlorine concentration(ppm) | 500 | 500 | 50 | 100 | 500 | 500 | 500 | 500 | 500 | 500 | 500 | 500 | 500 | 500 |
| | | pH | 7 | 7.5 | 6 | 6 | 6.5 | 6 | 6.5 | 6 | 6 | 6.5 | 6 | 6.8 | 11 | 6.5 |
| Germicidal property | | *Bacillus cerecus* | ○ | ○ | ○ | ○ | ○ | ○ | ○ | ○ | ○ | ○ | ○ | × | × | × |
| | | *Bacillus subtilis* | ○ | ○ | ○ | ○ | ○ | ○ | ○ | ○ | ○ | ○ | ○ | × | × | × |
| | | Mold | ○ | ○ | ○ | ○ | ○ | ○ | ○ | ○ | ○ | ○ | ○ | × | × | × |

*Amphitol 20N (made by Kao Corporation, effective amount 35 %)

**MCA-750 (made by Sakamoto Yakuhin Kogyo Co., Ltd.)

***LWA1570 (made by Mitsubishi-Kagaku Foods Corporation)

****Mydol 12 (made by Kao Corporation, effective amount 40 %)

Examples R12 to R20 and Comparative Example R4

An aqueous solution (2) compriseing components shown in Table R2 was diluted by adding ion exchanged water at a ratio shown in Table R2 to prepare a test-use aqueous solution (available chlorine concentration 200 ppm), and sealed into a sample bottle, and this was then stored in a thermostat at 40°C for two days. Here, among these aqueous solutions (2), those in examples R12 to R20 were obtained by mixing the same amount of a solution, which had been prepared by diluting a sodium hypochlorite aqueous solution (available chlorine concentration: 60000 ppm) with ion exchanged water to two times the final blending density, and a solution, which had been obtained by diluting a mixed solution prepared by mixing a surfactant and an organic acid at predetermined amounts with ion exchanged water to two times the final blended concentration. Two days later, the sample bottle was taken out, and the available chlorine concentration of the test-use aqueous solution was measured, the retention ratio (%) of available chlorine concentration was found through the following equation. Table R2 shows the results of the tests.

Retention ratio (%) = [(Available chlorine concentration after storage for two days at 40°C)/200] × 100.

Moreover, germicidal tests were carried out by using test-use aqueous solutions after the above-mentioned retention test. In other words, two kinds of spore-containing solutions that had been used in examples R1 to R11 and comparative examples of R1 to R3 were adjusted to $10^4$ cell/ml, and 0.1 ml of this was taken, and made in contact with 10 ml of the test-use aqueous solution (temperature: 25°C) after having been stored for ten seconds, and then gradually diluted by using an aqueous solution of 3.3 % sodium thiosulfate, and this was sprayed and applied to an SCD nutrient agar. This was then cultivated at 30°C for 48 hours, and the number of residual bacteria was found based upon the number of colonies through a conventional method. Table R2 shows the results of the tests.

Table R 2

| | | | Example | | | | | | | | | Comparative Example |
|---|---|---|---|---|---|---|---|---|---|---|---|---|
| | | | R12 | R13 | R14 | R15 | R16 | R17 | R18 | R19 | R20 | R4 |
| Blended components (weight %) of aqueous solution (2) | (A) | Sodium hypochlorite [Available chlorine concentration in aqueous solution (2)is shown in parentheses] | 1.05 (1) | 1.05 (1) | 1.05 (1) | 1.05 (1) | 1.05 (1) | 1.05 (1) | 1.05 (1) | 1.05 (1) | 1.05 (1) | 1.05 (1) |
| | (B) | Lauryldimethylamine oxide* | 1 | 1 | 1 | 1 | 1 | | | | | |
| | | Polyglyceim fatty acid ester** | | | | | | 0.5 | | | | |
| | | Sucrose fatty acid ester*** | | | | | | | 1 | | | |
| | | Alkylpolyglycoside**** | | | | | | | | 0.5 | | |
| | (C) | Succinic acid | 0.4 | | 0.85 | | | 1 | 0.85 | 1 | | |
| | | Fumaric acid | | 0.3 | | 0.65 | | | | | | |
| | Hydrochloric acid | | | | | | 0.5 | | | | p H adjustable amount | |
| | Water | | Blance | Balance | Balance | Balance | Balance | Balance | Balance | Balance | Balance | Balnce |
| | Total | | 100 | 100 | 100 | 100 | 100 | 100 | 100 | 100 | 100 | 100 |
| Test solution | | Dilution ratio of aqueous solution (2) | 50 | 50 | 50 | 50 | 50 | 50 | 50 | 50 | 50 | 50 |
| | | Available chlorine concentration (ppm) | 200 | 200 | 200 | 200 | 200 | 200 | 200 | 200 | 200 | 200 |
| | | pH | 7 | 7 | 6.5 | 6.5 | 6.5 | 6 | 6.5 | 6 | 6.5 | 11 |
| Retention stability | | Retention (%) of available chlorine | 80 | 45 | 75 | 40 | 38 | 83 | 85 | 75 | 42 | 93 |
| Number of residual bacteria (cfu/ml) | | *Bacillus cerecus* | <10 | 50 | <10 | 30 | 60 | <10 | <10 | <10 | 50 | $3.3\times10^3$ |
| | | *Bacillus subtilis* | <10 | 80 | <10 | 50 | 100 | <10 | <10 | <10 | 70 | $7.8\times10^3$ |

*Amphitol 20N (made by Kao Corporation, effective amount 35 %)

**MCA-750 (made by Sakamoto Yakuhin Kogyo Co., Ltd.)

***LWA1570 (made by Mitsubishi-Kagaku Foods Corporation)

****Mydol 12 (made by Kao Corporation, effective amount 40 %)

Examples S1 to S11 and Comparative Examples S1 to S3 (S1) Preparation of germicide composition

Each of the compositions of examples S1 to S5, S7 to S9 and comparative example S2 was obtained by mixing the same amounts of a solution, which had been prepared by mixing predetermined amounts of an aqueous solution of sodium hypochlorite (available chlorine concentration 60000 ppm) and (B) component or (G) component, and then diluting this by adding ion exchanged water to two times the final blended concentration, and a solution of succinic acid, which had been prepared by diluting succinic acid with ion exchanged water to two times the final blended concentration.

Moreover, of electrolytic oxidized water obtained by a diaphragm method, hypochlorous acid in water generated on the anode side (pH (25'C) 2.7, available chlorine concentration 50 ppm) was used, and adjusted to pH 11 by using 0.1 mol/L of aqueous solution of sodium hydroxide to obtain a composition represented by comparative example S3 of Table S1. Moreover, the above-mentioned hypochlorous acid in water was adjusted to pH 5 by using 1 mol/L of disodium succinate, and to this was added lauryldimethylamine oxide (the same composition as preferred embodiment S1) so as to have a concentration of 25 ppm; thus, the composition of preferred embodiment S6 shown in Table S1 was obtained. In the same manner, the compounds shown in Table S1 were used by the amounts shown in Table S1 to obtain the compositions of preferred embodiments S10 and S11.

Here, the available chlorine concentration of Table S1 was measured based upon "Iodometry" in JIS K-0101.

Test aqueous solutions, obtained by diluting these compositions to be set to available chlorine concentrations shown in Table S1 (in examples S6, S10, S11 and comparative example S-3, compositions, as they are, were applied), were used, and germicidal property tests were carried out by using the following method. Table S1 shows the results of the tests.

(S2) Evaluation of germicidal property

Approximately 300 g of soft cooked rice that had just been boiled was put into a plastic lunch box (170 mm × 120 mm × 43 mm), and cooled off at room temperature for two hours, and the cooked rice was taken out. A suspension of spores (approximately $10^9$ to $10^{10}$ cell/ml) (1 ml), obtained by subjecting spore-forming bacteria (*Bacillus cerecus* IFO13494) to a heating treatment through a conventional method, was uniformly sprayed into the lunch box. This lunch box was washed, sterilized and rinsed under the following conditions by using an automatic dish washer DW-2000R made by Sanyo Denki K.K., and the numbers of spores were compared with each other before and after the washing operation. Here, with respect to the numbers of spores before and after the washing operation, a predetermined area (50 mm × 50 mm) was wiped with swab, and this was dipped into 1 ml of sterilized water to form a suspension; then, this suspension was repeatedly subjected to 10-times dilution to prepare respective solutions, and 25μl of each solution was applied to a spore bacteria detection-use NGKG nutrient agar, and cultivated at 30°C for 48 hours; thereafter, the number of bacteria was measured. The number n of steps of dilution was set to 5, and the number of bacteria was calculated as the average value of three values that remained after the minimum value and the maximum values had been excluded.

<Washing Conditions>

Washing temperature: 60°C ± 2°C
Washing time: 15 seconds
Detergent: Axial made by Kao Corporation (not used in Comparative Example S1)
Detergent concentration: 0.15 %

<Germicidal Conditions>

Germicidal temperature: 60°C
Germicidal time: 10 seconds

<Rinsing Conditions>

Rinsing temperature: 60°C
Rinsing time: 5 seconds

Table S 1

| | | | Examples | | | | | | | | | | | Comparative example | | |
|---|---|---|---|---|---|---|---|---|---|---|---|---|---|---|---|---|
| | | | S1 | S2 | S3 | S4 | S5 | S6 | S7 | S8 | S9 | S10 | S11 | S1 | S2 | S3 |
| Germicide composition: Blended Components (weight %) | (A) | Sodium hypochlorite[(1)] | 1.05 (1) | 1.05 (1) | 1.05 (1) | 1.05 (1) | 1.05 (1) | | 1.05(1) | 1.05(1) | 1.05(1) | | | | 1.05 (1) | |
| | | Hypochlorous acid | | | | | | 50ppm | | | | 50ppm | 50ppm | | | 50ppm |
| | (B) | Lauryldimethylamine oxide[(2)] | 1 | 1 | | | 1 | 25ppm | | | | | | | | |
| | | Myristyldimethylamine oxide | | | 0.3 | | | | | | | | | | | |
| | | Benzalkonium chloride[(3)] | | | | 1 | | | | | | | | | | |
| | | Polyglycerin fatty acid ester[(4)] | | | | | | | 0.5 | | | 25ppm | | | | |
| | | Sucrose fatty acid ester[(5)] | | | | | | | | 1 | | | | | | |
| | | Alkylpolyglycoside[(6)] | | | | | | | | | 0.5 | | 25ppm | | | |
| | (C) | Succinic acid | 0.85 | 1 | 1.75 | 0.85 | 0.85 | | 1 | 0.85 | 1 | | | | | |
| | | Disodium succinate | | | | | | PH adjustable amount | | | | pH adjustable amount | pH adjustable amount | | | |
| | | Sodium hydroxide | | | | | | | | | | | | | | pH adjustable amount |
| | (G) | Polyoxyethylenelaurylether sulfate[(7)] | | | | | 0.5 | | | | | | | | | |
| | Water | | Balance | Balance | Balance | Balance | Balance | Balance | Balance | Balance | Balance | Balance | Balance | 100 | Balance | Balance |
| | Total | | 100 | 100 | 100 | 100 | 100 | 100 | 100 | 100 | 100 | 100 | 100 | 100 | 100 | 100 |
| Germicidal test | Test Solution | pH (20°C) | 6.5 | 6 | 5 | 6.5 | 6.5 | 5 | 6 | 6.5 | 6 | 5 | 5 | | 11 | 11 |
| | | Available chlorine concentration (ppm) | 100 | 100 | 50 | 100 | 100 | 50 | 100 | 100 | 100 | 50 | 50 | 0 | 200 | 50 |
| | Germicidal activity (Germicidal performance): Number of bacteria (cfu/cm²) | Before washing | $5.50\times10^{6}$ | $5.80\times10^{5}$ | $6.60\times10^{6}$ | $6.20\times10^{6}$ | $7.00\times10^{6}$ | $8.00\times10^{6}$ | $2.7\times10^{6}$ | $7.3\times10^{5}$ | $4.3\times10^{6}$ | $4.7\times10^{6}$ | $8.3\times10^{5}$ | $5.80\times10^{6}$ | $6.50\times10^{6}$ | $6.80\times10^{6}$ |
| | | After washing | $5.50\times10^{1}$ | $2.00\times10^{1}$ | $4.00\times10^{1}$ | $1.50\times10^{2}$ | $5.00\times10^{1}$ | $3.00\times10^{1}$ | $5.0\times10^{1}$ | $2.7\times10^{1}$ | $3.3\times10^{1}$ | $7.4\times10^{1}$ | $8.1\times10^{1}$ | $8.80\times10^{3}$ | $5.20\times10^{3}$ | $5.50\times10^{3}$ |

(1): The available chlorine concentration is given in parentheses.

(2): Amphitol 20N (made by Kao Corporation effective amount: 35 %) was used to set the effective concentration to the value indicated in Table S1.

(3): Sanisol C (made by Kao Corporation effective amount: 50 %) was used to set the effective concentration to the value indicated in Table S1.

(4): MCA-750 (made by Sakamoto Yakuhin Kogyo Co., Ltd.) was used to set the effective concentration to the value indicated in Table S1.

(5): LWA1570 (made by Mitsubishi-Kagaku Foods Corporation) was used to set the effective concentration to the value indicated in Table S1.

(6): Mydol 12 (made by Kao Corporation effective amount: 40 %) was used to set the effective concentration to the value indicated in Table S1.

(7): Emal 20C (made by Kao Corporation effective amount: 25 %) was used to set the effective concentration to the value indicated in Table S1.

Examples T1 to T11 and Comparative Examples T1 to T3

(T1) Preparation of germicide composition

The compositions of examples T1 to T5, T7 to T9 and comparative example T2 of Table T1 were obtained in the same manner as example (S1). In comparative example T1, sterile water was used.

Moreover, in the same manner as example S1, hypochlorous acid in water generated on the anode side as described earlier was used to adjust to pH 11, thereby obtaining compositions of comparative example T3 in Table T1. Furthermore, after the above-mentioned hypochlorous acid in water had been adjusted to pH5 in the same manner as example (S1), to this was added lauryldimethylamine oxide (the same as example T1) to have a concentration of 25 ppm, thereby obtaining the composition of example T6 in Table T1. In the same manner, compositions of examples T10 and T11 were obtained. Here, in Table T1, the available chlorine concentration was measured based upon the above-mentioned "Iodometry".

Test aqueous solutions, obtained by diluting these compositions to be set to available chlorine concentrations shown in Table T1 (in examples T6, T10, T11 and comparative example T3, compositions, as they are, were applied), were used, and germicidal property tests were carried out by using the following method. Table T1 shows the results of the tests.

(T2) Evaluation of germicidal property

Cotton broad (material cloth, undyed cloth) was cut into pieces, each having a size of 10 cm × 24 cm, and this was subjected to an autoclave sterilizing process (121°C, 15 minutes), and cooled and dried in a clean bench; thus, test cloths were prepared. A suspension of spores (approximately $10^7$ to $10^8$ cell/ml) (1 ml), obtained by subjecting spore-forming bacteria (Bacillus subtilis ATCC6633) to a heating treatment through a conventional method, was further diluted by sterilized water to 100 ml to form a bacteria solution (approximately $10^5$ to $10^6$ cell/ml), and 10 sheets of the above-mentioned cloths (weight of cloths: 25 g, bath ratio 1/4) were immersed into the solution, and processed at 25°C for 10 minutes. Next, this was subjected to centrifugal dehydration for 30 seconds to obtain 75 g of moistened cloths. Then, 0.5 ml of liquid portions of these moistened cloths was taken to prepare a bacteria-number counting sample prior to a germicidal process.

Next, the above-mentioned moistened cloths were immersed into 100 g of each of the compositions shown in Table T1, and processed at 25°C for 10 minutes. Thereafter, this was processed in sodium thiosulfate (hypo), and subjected to centrifugal dehydration for 30 seconds, thereby obtaining 75 g of moistened cloths after germicidal process. Then, 0.5 ml of liquid portions of these moistened cloths was taken to prepare a bacteria-number counting sample after the germicidal process.

Each of the measuring samples was successively diluted by adding sterilized water thereto step by step, and this was cultivated on an SCD nutrient agar (made by Nissui Pharmaceutical Co., Ltd. at 30°C for 48 hours; thereafter, the number of bacteria was measured through a conventional method. Table T1 shows the results of the tests.

Table T 1

| | | | | | Examples | | | | | | | | | | | Comparative example | | |
|---|---|---|---|---|---|---|---|---|---|---|---|---|---|---|---|---|---|---|
| | | | | | T1 | T2 | T3 | T4 | T5 | T6 | T7 | T8 | T9 | T10 | T11 | T1 | T2 | T3 |
| Germicide composition | Blended Components(Weight %) | (A) | Sodium hypochlorite[(1)] | | 1.05 (1) | 1.05 (1) | 1.05 (1) | 1.05 (1) | 1.05 (1) | | 1.05 (1) | 1.05 (1) | 1.05 (1) | | | | 1.05 (1) | |
| | | | Hypochlorous acid | | | | | | | 50ppm | | | | 50ppm | 50ppm | | | 50ppm |
| | | (B) | Lauryldimethylamine oxide[(2)] | | 1 | 1 | | | 1 | 25ppm | | | | | | | | |
| | | | Myristyldimethylamine oxide | | | | 0.3 | | | | | | | | | | | |
| | | | Benzalkonium chloride[(3)] | | | | | 1 | | | | | | | | | | |
| | | | Polyglycerin fatty acid ester[(4)] | | | | | | | | 0.5 | | | 25ppm | | | | |
| | | | Sucrose fatty acid ester[(5)] | | | | | | | | | 1 | | | | | | |
| | | | Alkylpolyglycoside[(6)] | | | | | | | | | | 0.5 | | 25ppm | | | |
| | | (C) | Succinic acid | | 0.85 | 1 | 1.75 | 0.85 | 0.85 | | 1 | 0.85 | 1 | | | | | |
| | | | Disodium succinate | | | | | | | pH adjustable amount | | | | pH adjustable amount | pH adjustable amount | | | |
| | | | Sodium hydroxide | | | | | | | | | | | | | | | pH adjustable amount |
| | | (G) | Polyoxyethylenelaurylether saulfate[(7)] | | | | | | 0.5 | | | | | | | | | |
| | | Water | | | Balance | Balance | Balance | Balance | Balance | Balance | Balance | Balance | Balance | Balance | Balance | Balance | Balance | Balance |
| | | Total | | | 100 | 100 | 100 | 100 | 100 | 100 | 100 | 100 | 100 | 100 | 100 | 100 | 100 | 100 |
| Germicidal test | Test solution | pH(20°C) | | | 6.5 | 6 | 5 | 6.5 | 6.5 | 5 | 250 | 250 | 250 | 250 | 250 | 6.5 | 11 | 11 |
| | | Available chlorine concentration (ppm) | | | 250 | 250 | 250 | 250 | 250 | 50 | 6 | 6.5 | 6 | 5 | 5 | 0 | 250 | 50 |
| | Germicidal activity ( Germicidal performance ) | Number of bacteris (cfu/cm²) | Before washing | | $4.00 \times 10^5$ | $3.00 \times 10^5$ | $3.30 \times 10^5$ | $3.80 \times 10^5$ | $3.60 \times 10^5$ | $3.50 \times 10^5$ | $3.7 \times 10^5$ | $3.8 \times 10^5$ | $3.5 \times 10^5$ | $3.3 \times 10^5$ | $3.5 \times 10^5$ | $3.50 \times 10^5$ | $4.10 \times 10^5$ | $3.10 \times 10^5$ |
| | | | After washing | | $2.30 \times 10^2$ | $1.00 \times 10^1$ | $2.00 \times 10^1$ | $8.50 \times 10^2$ | $2.20 \times 10^2$ | $1.00 \times 10^1$ | $1.5 \times 10^1$ | $2.2 \times 10^1$ | $1.2 \times 10^1$ | $1.3 \times 10^1$ | $1.1 \times 10^1$ | $9.00 \times 10^4$ | $5.00 \times 10^4$ | $7.20 \times 10^4$ |

(1): The available chlorine concentration is given in parentheses.

(2): Amphitol 20N (made by Kao Corporation effective amount: 35 %) was used to set the effective concentration to the value indicated in Table T1.

(3): Sanisol C (made by Kao Corporation effective amount: 50 %) was used to set the effective concentration to the value indicated in Table T1.

(4): MCA-750 (made by Sakamoto Yakuhin Kogyo Co., Ltd.) was used to set the effective concentration to the value indicated in Table T1.

(5): LWA1570 (made by Mitsubishi-Kagaku Foods Corporation) was used to set the effective concentration to the value indicated in Table T1.

(6): Mydol 12 (made by Kao Corporation effective amount: 40 %) was used to set the effective concentration to the value indicated in Table T1.

(7): Emal 20C (made by Kao Corporation effective amount: 25 %) was used to set the effective concentration to the value indicated in Table T1.

Examples U1 to U1 and Comparative Examples U1 to U4

(U1) Preparation of germicidal washing agent compositions used for perishable foodstuff

The compositions of examples U1 to U5, U7 to U9 and comparative examples U1 to U3 of Table U1 were obtained in the same manner as example (S1).

Moreover, in the same manner as example (S1), hypochlorous acid in water generated on the anode side as described earlier was used to adjust to pH 11, thereby obtaining compositions of comparative example U4 in Table U1. Furthermore, after the above-mentioned hypochlorous acid in water had been adjusted to pH5 in the same manner as example (S1), to this was added lauryldimethylamine oxide (the same as example U1) to have a concentration of 25 ppm, thereby obtaining the composition of example U6 in Table U1. In the same manner, compositions of examples U10 and U11 were obtained.

Here, in Table U1, the available chlorine concentration was measured based upon the above-mentioned "Iodometry".

Test aqueous solutions, obtained by diluting these compositions to be set to available chlorine concentrations shown in Table U1 (in examples U6, U10, U11 and comparative example U4, compositions, as they are, were applied), were used, and germicidal property tests were carried out by using the following method. Table U1 shows the results of the tests.

(U2) Evaluation of germ-eliminating and germicidal properties

Two sheets of outer shells of commercially available cabbage were pelt off, and divided into four portions, and these were shredded to weigh approximately 100 g. These were put into a 1-liter beaker in a manner so as to allow the pieces to separate from each other. Each of the compositions of Table U1 was added thereto so as to have a bath ratio of 10, and these were washed for 3 minutes, and subjected to rinsing processes for 30 seconds twice (1 liter of tap water) with water being held. Any of these processes were carried out at room temperature. After the final rinsing process, the cabbage was homogenized to prepare a bacteria-number counting sample. The number of N was set to 5, and the number of general alive bacteria per 1 g of cabbage was measured in conformity with the food sanitary inspection guidance. The germ-removing and germicidal properties were calculated based upon the following equation:

$$\text{Germ-removing and germicidal rate (\%)} = [(A - B)/A] \times 100$$

A; number of bacteria before washing (number/1g of cabbage)
B; number of bacteria after washing (number/1g of cabbage)

Table U 1

| | | | Example | | | | | | | | | | | Comparative example | | | |
|---|---|---|---|---|---|---|---|---|---|---|---|---|---|---|---|---|---|
| | | | U1 | U2 | U3 | U4 | U5 | U6 | U7 | U8 | U9 | U10 | U11 | U1 | U2 | U3 | U4 |
| Germicidal washing agent composition | Blended components(weight %) (A) | Sodium hypochlorite[1] | 1.05 (1) | 1.05 (1) | 1.05 (1) | 1.05 (1) | 1.05 (1) | | 1.05 (1) | 1.05 (1) | 1.05 (1) | | | 1.05 (1) | | 1.05 (1) | |
| | (A) | Hypochlorous acid | | | | | | 50 ppm | | | | 50 ppm | 50 ppm | | | | 50 ppm |
| | (B) | Lauryldimethylamine oxide (2) | 1 | 1 | | | 1 | 25 ppm | | | | | | | 1 | | |
| | (B) | Myristyldimethylamine oxide | | | 0.3 | | | | | | | | | | | | |
| | (B) | Benzalkonium chloride[3] | | | | 1 | | | | | | | | | | | |
| | (B) | Polyglycerin fatty acid ester(4) | | | | | | | 0.5 | | | 25 ppm | | | | | |
| | (B) | Sucrose fatty acid ester(5) | | | | | | | | 1 | | | | | | | |
| | (B) | Alkylpolyglycoside(6) | | | | | | | | | 0.5 | | 25 ppm | | | | |
| | (C) | Succinic acid | 0.85 | 1 | 1.75 | 0.85 | 0.85 | | 1 | 0.85 | 1 | | | | 1 | 0.85 | |
| | (C) | Disodium succinate | | | | | | pH adjustable amount | | | | pH adjustable amount | pH adjustable amount | | | | |
| | (C) | Sodium hydroxide | | | | | | | | | | | | | | | pH adjustable amount |
| | (G) | Polyoxyethylenelaurylether sulfate[7] | | | | | 0.5 | | | | | | | | | | |
| | Water | | Balance | Balance | Balance | Balance | Balance | Balance | Balance | Balance | Balance | Balance | Balance | Balance | Balance | Balance | Balance |
| | Total | | 100 | 100 | 100 | 100 | 100 | 100 | 100 | 100 | 100 | 100 | 100 | 100 | 100 | 100 | 100 |
| Germ-removing and germicidal property test | Test solution | pH(20°C) | 6.5 | 6 | 5 | 6.5 | 6.5 | 5 | 6 | 6.5 | 6 | 5 | 5 | 11 | 3.5 | 6.5 | 11 |
| | Test solution | Available Chlorine concentration(ppm) | 200 | 200 | 200 | 200 | 200 | 50 | 200 | 200 | 200 | 50 | 50 | 200 | 0 | 200 | 50 |
| | Germ-removing and germicidal rate (%) | | over 99 | over 99 | over 99 | over 99 | over 99 | over 99 | over 99 | over 99 | over 99 | over 99 | over 99 | 91 | 70 | 93 | 85 |

(1): The available chlorine concentration is given in parentheses.

(2): Amphitol 20N (made by Kao Corporation effective amount: 35 %) was used to set the effective concentration to the value indicated in Table U1.

(3): Sanisol C (made by Kao Corporation effective amount: 50 %) was used to set the effective concentration to the value indicated in Table U1.

(4): MCA-750 (made by Sakamoto Yakuhin Kogyo Co., Ltd.) was used to set the effective concentration to the value indicated in Table U1.

(5): LWA1570 (made by Mitsubishi-Kagaku Foods Corporation) was used to set the effective concentration to the value indicated in Table U1.

(6): Mydol 12 (made by Kao Corporation effective amount: 40 %) was used to set the effective concentration to the value indicated in Table U1.

(7): Emal 20C (made by Kao Corporation effective amount: 25 %) was used to set the effective concentration to the value indicated in Table U1.

Examples V1 to V11 and Comparative Examples V1 to V4

(V1) Preparation of virucide compositions

The compositions of examples V1 to V5, V7 to V9 and comparative examples V1 to V3 of Table V1 were obtained in the same manner as example (S1).

Moreover, in the same manner as example (S1), hypochlorous acid in water generated on the anode side as described earlier was used to adjust to pH 11, thereby obtaining compositions of comparative example V4 in Table V1. Furthermore, after the above-mentioned hypochlorous acid in water had been adjusted to pH5 in the same manner as example (S1), lauryldimethylamine oxide (the same as example V1) was added thereto to have a concentration of 25 ppm, thereby obtaining the composition of example V6 in Table V1. In the same manner, compositions of examples V10 and V11 were obtained.

Here, in Table V1, the available chlorine concentration was measured based upon the above-mentioned "Iodometry".

Test aqueous solutions, obtained by diluting these compositions to be set to available chlorine concentrations shown in Table V1 (in examples V6, V10, V11 and comparative example V4, compositions, as they are, were applied), were used, and germicidal property tests were carried out by using the following method. Table V1 shows the results of the tests.

(V2) Evaluation of virucide property

<Subject virus>

(i) poliomyelitis virus: poliomyelitis virus type 3, vaccine stock (Sabin stock)
(ii) herpes simplex virus: HF stock

With respect to the measurements of virus propagation and infection valence, FL tissues were used.

<Testing method>

Each of virucide compositions (50µl) of Table V1 and 50µlm of virus solution were mixed. This was left still at 20°C for 30 seconds, and 50µl of 2 % sodium thiosulfate was added thereto. This mixed solution was diluted to 10 times step by step, and the respective solutions were subjected to measurements on the virus infection valence. Here, with respect to virus control group, an aqueous solution of 50µL of 2 % sodium thiosulfate was added to 50µL of each of the compositions, and after this had been left for 30 minutes, 50µL of virus solution was added thereto.

Table V 1

| | | | Example | | | | | | | | | | | Comparative example | | | |
|---|---|---|---|---|---|---|---|---|---|---|---|---|---|---|---|---|---|
| | | | V1 | V2 | V3 | V4 | V5 | V6 | V7 | V8 | V9 | V10 | V11 | V1 | V2 | V3 | V4 |
| Virucide composition | Blended components (weight %) (A) | Sodium hypochlorite(1) | 1.05 (1) | 1.05 (1) | 1.05 (1) | 1.05 (1) | 1.05 (1) | | 1.05 (1) | 1.05 (1) | 1.05 (1) | | | 1.05 (1) | 1.05 (1) | 1.05 (1) | |
| Virucide composition | Blended components (weight %) (A) | Hypochlorous acid | | | | | | 50ppm | | | | 50ppm | 50ppm | | | | 50ppm |
| Virucide composition | Blended components (weight %) (B) | Lauryldimethylamine oxide(2) | 1 | 1 | | | 1 | 25ppm | | | | | | | | | |
| Virucide composition | Blended components (weight %) (B) | Myristyldimethylamine oxide | | | 0.3 | | | | | | | | | | | | |
| Virucide composition | Blended components (weight %) (B) | Benzalkonium chloride(3) | | | | 1 | | | | | | | | | | | |
| Virucide composition | Blended components (weight %) (B) | Polyglycerin fatty acid ester (4) | | | | | | | 0.5 | | | 25ppm | | | | | |
| Virucide composition | Blended components (weight %) (B) | Sucrose fatty acid ester (5) | | | | | | | | 1 | | | | | | | |
| Virucide composition | Blended components (weight %) (B) | Alkylpolyglycoside (6) | | | | | | | | | 0.75 | | 25ppm | | | | |
| Virucide composition | Blended components (weight %) (C) | Succinic acid | 0.85 | 1 | 1.75 | 0.85 | 0.85 | | 1 | 1.25 | 1 | | | | | | |
| Virucide composition | Blended components (weight %) (C) | Disodium succinate | | | | | | pH adjustable amount | | | | pH adjustable amount | pH adjustable amount | | 0.85 | | |
| Virucide composition | Blended components (weight %) (C) | Sodium hydroxide | | | | | | | | | | | | | | | pH adjustable amount |
| Virucide composition | Blended components (weight %) (G) | Polyoxyethylenelaurylether sulfate (7) | | | | | 0.5 | | | | | | | | | | |
| Virucide composition | Blended components (weight %) (G) | Polyoxyethylenelauryl ether(8) | | | | | | | | | | | | | | 1 | |
| Virucide composition | Blended components (weight %) | Water | Balance | Balance | Balance | Balance | Balance | Balance | Balance | Balance | Balance | Balance | Balance | Balance | Balance | Balance | Balance |
| Virucide composition | Blended components (weight %) | Total | 100 | 100 | 100 | 100 | 100 | 100 | 100 | 100 | 100 | 100 | 100 | 100 | 100 | 100 | 100 |
| Virucide test | Test solution | pH(20°C) | 6.5 | 6 | 5 | 6.5 | 6.5 | 5 | 6 | 5.7 | 6 | 5 | 5 | 11 | 6.5 | 11 | 11 |
| Virucide test | Test solution | Available chlorine concentration (ppm) | 200 | 200 | 200 | 200 | 200 | 50 | 200 | 200 | 200 | 50 | 50 | 200 | 200 | 200 | 50 |
| Virucide test | poliomyelitis virus | Virus infection valence ($log_{10}TCID_{50}$/ml) | less than1.5 | less than1.5 | less than1.5 | less than1.5 | less than1.5 | less than1.5 | less than1.5 | less than1.5 | less than1.5 | less than1.5 | less than1.5 | 2.0 | 1.7 | 2.0 | 3.5 |
| Virucide test | poliomyelitis virus | Virus control | 7.5 | 7.5 | 7.5 | 7.5 | 7.5 | 7.5 | 7.5 | 7.5 | 7.5 | 7.5 | 7.5 | 7.5 | 7.5 | 7.5 | 7.5 |
| Virucide test | Herpes simplex virus | Virus infection valence ($log_{10}TCID_{50}$/ml) | less than 1.9 | less than 1.9 | less than 1.9 | less than 1.9 | less than 1.9 | less than 1.9 | less than 1.9 | less than 1.9 | less than 1.9 | less than 1.9 | less than 1.9 | 2.5 | 2.2 | 2.5 | 4.0 |
| Virucide test | Herpes simplex virus | Virus control | 6.7 | 6.7 | 6.7 | 6.7 | 6.7 | 6.7 | 6.7 | 6.7 | 6.7 | 6.7 | 6.7 | 6.7 | 6.7 | 6.7 | 6.7 |

(1): The available chlorine concentration is given in parentheses.

(2): Amphitol 20N (made by Kao Corporation effective amount: 35 %) was used to set the effective concentration to the value indicated in Table V1.

(3): Sanisol C (made by Kao Corporation effective amount: 50 %) was used to set the effective concentration to the value indicated in Table V1.

(4): MCA-750 (made by Sakamoto Yakuhin Kogyo Co., Ltd.) was used to set the effective concentration to the value indicated in Table V1.

(5): LWA1570 (made by Mitsubishi-Kagaku Foods Corporation) was used to set the effective concentration to the value indicated in Table V1.

(6): Mydol 12 (made by Kao Corporation effective amount: 40 %) was used to set the effective concentration to the value indicated in Table V1.

(7): Emal 20C (made by Kao Corporation effective amount: 25 %) was used to set the effective concentration to the value indicated in Table V1.

(8): Emulgen 106 (made by Kao Corporation) was used to set the effective concentration to the value indicated in Table V1.

Examples W1 to W10 and Comparative Examples W1 to W3

The following tests were carried out by using compositions having components shown in Table W1. Table W1 shows the results of the tests. Here, in Table W1, the available chlorine concentration was measured based upon the above-mentioned "Iodometry". Here, each of the compositions was obtained by mixing the same amounts of a solution, which had been prepared by mixing predetermined amounts of an aqueous solution of sodium hypochlorite (available chlorine concentration 60000 ppm) and (B) component or (F) component, and then diluting this by adding ion exchanged water to two times the final blended concentration, and a solution of succinic acid, which had been prepared by diluting succinic acid with ion exchanged water to two times the final blended concentration.

In each of the following tests, each of the compositions of Table W1 was diluted to have the available chlorine concentration of Table W1 to prepare a sample aqueous solution, and this was used. (W1) Mold-eliminating test

(W1-1) Preparation of mold-stained plates

Mold (fungi: Aspergillus niger IFO6341) was cultivated as test-subject germ in PDA culture medium at 25°C for 7 days. The resulting mycobiont was uniformed by using a glass bead sterilizing method, and foreign matters were removed therefrom by sterilizing gauze, thereby obtaining a solution containing bacteria (approximately, $10^5$ cell/ml). Then, 0.5 ml of this solution containing bacteria was inoculated onto an ABS resin plate (longitudinal length 76 mm × lateral length 26 mm × thickness 1 mm), and cultivated at 30°C, 90 %RH for three days, thereby obtaining a mold-stained plate.

(W1-2) Detergency on mold stain

The composition made of a component of Table W1 was further diluted by adding ion exchanged water to prepare an aqueous solution (temperature: 25°C), and 1 ml of this was inoculated onto the mold-stained plate, and 15 minutes later, the resulting plate was washed, and dried in air; then, the conditions of the plate were visually observed, and evaluated based upon the following 5 grades. Table W1 shows the results of the tests.

5 ... Mold stains were completely removed.
4 ... Almost all the mold stains were removed.
3 ... Virtually half the mold stains were removed.
2 ... Hardly any mold stains were removed.
1 ... None of mold stains were removed.

(W1 - 3) Germicidal property

A predetermined area (10 mm × 10 mm) of the plate that had been subjected to the inoculation of the diluted aqueous solution (temperature: 25°C) in the above-mentioned (W1-2), and washed and dried in air was wiped with sterilized swab, and this swab was dipped into 1 ml of sterilized water so that the accretion was suspended therein; thus, 0.1 ml of the suspension was inoculated onto a PDA culture medium (containing 3.3 % of sodium thiosulfate). This was cultivated at 25°C for 7 days, and visually observed as to whether or not any mold had grown; thus, when no growth of the mold growth observed, this case was evaluated as "◎ ". and when any growth thereof was observed, this case was evaluated as "×". Table W1 shows the results of the tests.

Table W 1

| | | | Example | | | | | | | | | | Comparative example | | |
|---|---|---|---|---|---|---|---|---|---|---|---|---|---|---|---|
| | | | W1 | W2 | W3 | W4 | W5 | W6 | W7 | W8 | W9 | W10 | W1 | W2 | W3 |
| Mold-removing agent composition: Blended components (weight %) | (A) | Sodium hypochlorite[1] | 1.05 (1) | 1.05 (1) | 1.05 (1) | 1.05 (1) | 1.05 (1) | 1.05(1) | 1.05(1) | 1.05(1) | 1.05(1) | 1.05(1) | 1.05 (1) | 1.05 (1) | 1.05 (1) |
| | (B) | Lauryldimethylamine oxide[2] | 1 | 1 | | | 1 | | | | | | | | |
| | (B) | Myristyldimethylamine oxide* | | | 0.3 | | | | | | | | | | |
| | (B) | Benzalkonium chloride[3] | | | | 1 | | | | | | | | | |
| | (B) | Polyglycerin fatty acid ester(4) | | | | | | 1 | 0.5 | | | | | | |
| | (B) | Sucrose fatty acid ester (5) | | | | | | | | 1 | | | | | |
| | (B) | Alkylpolyglycoside (6)* | | | | | | | | | 1 | 0.5 | | | |
| | (C) | Succinic acid | 0.85 | 1 | 1.75 | 0.85 | 0.85 | 0.85 | 1 | 1 | 0.85 | 1 | | 0.85 | |
| | (F) | Polyoxyethylenelaurylether sulfate[7] | | | | | 0.5 | | | | | | | | |
| | | Polyoxyethylenelauryl ether[8] | | | | | | | | | | | | | 1 |
| | | Water | Balance | Balance | Balance | Balance | Balance | Balance | Balance | Balance | Balance | Balance | Balance | Balance | Balance |
| | | Total | 100 | 100 | 100 | 100 | 100 | 100 | 100 | 100 | 100 | 100 | 100 | 100 | 100 |
| Mold-removing test | Test solution | Available chlorine concentration (ppm) | 200 | 200 | 200 | 200 | 200 | 200 | 200 | 200 | 200 | 200 | 200 | 200 | 200 |
| | Test solution | pH (20°C) | 6.5 | 6 | 5 | 6.5 | 6.5 | 6.5 | 6 | 6 | 6.5 | 6 | 11 | 6.5 | 11 |
| | | Mold-stain detergency | 5 | 5 | 5 | 5 | 5 | 5 | 5 | 5 | 5 | 5 | 3 | 3 | 3 |
| | | Germicidal activity (Germicidal performance) | ◎ | ◎ | ◎ | ◎ | ◎ | ○ | ○ | ○ | ○ | ○ | × | × | × |

(1): The available chlorine concentration is given in parentheses.

(2): Amphitol 20N (made by Kao Corporation effective amount: 35 %) was used to set the effective concentration to the value indicated in Table W1.

(3): Sanisol C (made by Kao Corporation effective amount: 50 %) was used to set the effective concentration to the value indicated in Table W1.

(4): MCA-750 (made by Sakamoto Yakuhin Kogyo Co., Ltd.) was used to set the effective concentration to the value indicated in Table W1.

(5): LWA1570 (made by Mitsubishi-Kagaku Foods Corporation) was used to set the effective concentration to the value indicated in Table W1.

(6): Mydol 12 (made by Kao Corporation effective amount: 40 %) was used to set the effective concentration to the value indicated in Table W1.

(7): Emal 20C (made by Kao Corporation effective amount: 25 %) was used to set the effective concentration to the value indicated in Table W1.

(8): Emulgen 106 (made by Kao Corporation) was used to set the effective concentration to the value indicated in Table W1.

Examples W11 to W14 and Comparative Example W4

Of electrolytic oxidized water obtained by a diaphragm method, hypochlorous acid in water generated on the anode side (pH (25°C) 2.7, available chlorine concentration 50 ppm) was used, and adjusted to pH 11 by using 0.1 mol/L of aqueous solution of sodium hydroxide to obtain a composition represented by comparative example W4 of Table W2. Moreover, the above-mentioned hypochlorous acid in water was adjusted to pH 5 by using 1 mol/L of disodium succinate, and lauryldimethylamine oxide (the same composition as example W1) was added thereto so as to have a concentration of 25 ppm; thus, the composition of example W11 of Table W2 was obtained. In the same manner, the compounds of examples W12 to W14 were also obtained. In the same manner as example W1, tests were carried out on mold-stain detergency and germicidal property by using these compositions. Table W2 shows the results of the tests.

Table W 2

| | | Example | | | | Comparative example |
|---|---|---|---|---|---|---|
| | | W11 | W12 | W13 | W14 | W4 |
| Blended components (A) | Hypochlorous acid | 50ppm | 50ppm | 50ppm | 50ppm | 50ppm |
| Blended components (B) | Lauryldimethylamine oxide* | 25ppm | | | | — |
| | Polyglycerin fatty acid ester** | | 25ppm | | | |
| | Sucrose fatty acid ester*** | | | 25ppm | | |
| | Alkylpolyglycoside**** | | | | 25ppm | |
| Blended components (C) | Disodium succinate | pH adjustable amount | pH adjustable amount | pH adjustable amount | pH adjustable amount | — |
| | Sodium hydroxide | — | | | | pH adjustable amount |
| pH(20°C) | | 5 | 5 | 5 | 5 | 11 |
| Mold-stain detergency | | 4 | 4 | 4 | 4 | 1 |
| Germicidal activity (germicidal performance) | | ◎ | ○ | ○ | ○ | × |

*Amphitol 20N (made by Kao Corporation, effective amount 35 %)

**MCA-750 (made by Sakamoto Yakuhin Kogyo Co., Ltd.)

***LWA1570 (made by Mitsubishi-Kagaku Foods Corporation)

****Mydol 12 (made by Kao Corporation, effective amount 40 %)

Examples X1 to X8 and Comparative Examples X1 to X3

By using each of the compositions made from components listed in Table X1, the following tests were' carried out. Table X1 shows the results of the tests. Here, in Table X1, the available chlorine concentration was measured based upon the above-mentioned "Iodometry".

The respective compositions were obtained in the same manner as examples W1 to W10 and comparative examples W1 to W3.

(X1) Detergency

Model stains of oil stain and protein stain were respectively prepared, and the respective washing properties were evaluated by reanutstest modified method. Here, in any of these tests, test solutions, obtained by diluting the compositions of Table X1 so as to be set to the available chlorine concentrations shown in Table X1, were used.

(X1-1) Oil stain detergency

Fat and oil (20 g), formed by mixing beef tallow and soybean oil at a volume ratio of 1:1, 0.25 g of mono-olein and 0.1 g of oil red were dissolved in 60 ml of chloroform to prepare an oil stain solution. A set of six sheets of clean slide glass was prepared and the mass of each of the sheets was measured to the unit of 1 mg. The sheets of slide glass were immersed into the oil stain solution at 25 ± 1°C sheet by sheet for approximately 2 seconds up to approximately 55 mm so that oil stain was allowed to adhere thereto, and the sheets of glass were then taken out. The excessive portion of the oil stain adhering to the lower portion of the sheet of slide glass was absorbed by clean cloth such as gauze and filter paper so that the adhesion of oil stain was maintained evenly; then, this was dried in air at 25 ± 1°C and the mass thereof was measured. These model stain glass plates were used in tests after they had been left and dried for a period from not less than one hour to not more than two hours. In this case, the amount of adhesion of oil stain per six sheets of model stain glass plates was set to 0.140 ± 0.010 g.

Six sets of these model stain glass plates were washed at 25 ± 2°C for 5 minutes by using Leenertsmodified washing machine, and then rinsed by ion exchanged water at 25 ± 2°C for 30 seconds. The glass plates that had been subjected to the rinsing process were dried in air for one whole-day. The evaluation of detergency was carried out based upon the weights of the model stain glass plates before and after the washing process. In other words, the difference in weight before and after the washing process was found so that the detergency (%) was calculated based upon the following equation.

$$\text{Detergency (\%)} = (\text{Weight before washing} - \text{Weight after washing}) / \text{Amount of stain adhesion} \times 100$$

The detergencies were found on the respective six sheets of slide glass, and the average value of the four sheets that remained after the maximum value and the minimum value had been excluded was determined as the detergency of the corresponding composition.

(X1 - 2) Protein stain detergency

Defatted milk (20 g) was diluted and dissolved in ion exchanged water at 60°C to form a protein stain solution of the total weight of 100 g.
Sheets of clean slide glass were immersed into the protein stain solution at 25 ± 1°C sheet by sheet for approximately 2 seconds up to approximately 55 mm so that protein stain was allowed to adhere thereto, and the sheets of glass were then taken out. The excessive portion of the protein stain adhering to the lower portion of the sheet of slide glass was absorbed by clean cloth such as gauze and filter paper so that the adhesion of protein stain was maintained evenly; then, this was dried in air at 25 ± 1°C. This process was repeated again, and after stain on one face had been completely removed, this was dried in air, and denatured at 110°C for one hour to prepare a test piece. These test pieces were used in tests within a period from not less than 12 hours to not more than 24 hours. The test pieces were washed at 25 ± 2°C for 5 minutes by using Leenertsmodified washing machine, and then rinsed by ion exchanged water at 25 ± 2°C for 30 seconds. The test pieces that had been subjected to the rinsing process were dried at 70°C for 30 minutes, and colored by a 1 weight % solution of erythrosine; then, the colored area ($S_1$) was measured by photographic decision so that, based upon the initial (before washing) protein stain adhesion area ($S_0$), the detergency (%) was calculated from the following equation.

$$\text{Detergency (\%)} = (S_0 - S_1) / S_0 \times 100$$

The detergency was found on the respective six sheets of slide glass, and the average value of the four sheets that remained after the maximum value and the minimum value had been excluded was determined as the detergency of the corresponding composition.

(X2) Germicidal property

(X2-1) Sporecide test

(1) *Bacillus subtilis* ATCC6633, which is spore-forming bacteria, was used, and a loop of bacteria, which had been preliminarily cultivated in an SCD agar (made by Nissui Pharmaceutical Co., Ltd.), was suspended in 1 ml of sterilized water, and subjected to a heating treatment at 65°C for 30 minutes, and this was then washed twice while being subjected to centrifugal separation; thus, the resulting bacteria ($10^5$ cell/ml) were used in the test.

Then, 0.1 ml of this sample spore-containing solution was taken, and made in contact with 10 ml of a test-use aqueous solution (temperature: 25 °C) for 3 minutes which had been prepared by further diluting aqueous solution composed of components listed in table X1 with sterilized ion exchanged water. Within 10 seconds, 50μl of this bacteria-contact solution was sampled and inoculated onto a micro-plate (which was the same as that used in [R1]) containing 0.2 ml of post-cultivation-use SCDLP medium (containing 3.3 % of sodium thiosulfate). This was cultivated at 30'C for 48 hours, and the growth of the bacteria was visually observed; thus, it was determined whether or not the bacteria had grown up on the micro-plate. Then, the minimum dilution rate (minimum germicidal available chlorine concentration) at which no growth of bacteria was observed (which indicates 100 % germicidal property) was found. Here, the available chlorine concentration was measured based upon the aforementioned "Iodometry".

(X2-2) Moldcide test

A solution containing mold was obtained in the same manner as example [R2] (approximately, $10^5$ cell/ml). Then, 0.1 ml of this solution was taken, and inoculated into 10 ml of an aqueous solution (temperature: 25 °C) which had contained each composition made from components shown in Table X1 and had been further diluted by sterilized ion exchanged water, for 3 minutes at room temperature. Within 10 seconds, 0.1 ml of this solution was sampled, and inoculated onto post-cultivation-use PDA medium (containing 3.3 % of sodium thiosulfate). This was cultivated at 25°C for 7 days, and the growth of the mold was visually observed, and evaluated in the same manner as described above.

Table X 1

| | | | | Example | | | | | | | | Comparative example | | |
|---|---|---|---|---|---|---|---|---|---|---|---|---|---|---|
| | | | | X1 | X2 | X3 | X4 | X5 | X6 | X7 | X8 | X1 | X2 | X3 |
| Germicidal washing agent composition | Blended components(weight %) | (A) | Sodium hypochlorite(1) | 1.05 (1) | 1.05 (1) | 1.05 (1) | 1.05 (1) | 1.05 (1) | 1.05 (1) | 1.05 (1) | 1.05 (1) | 1.05 (1) | 1.05 (1) | 1.05 (1) |
| | | (B) | Lauryldimethylamine oxide(2) | 1 | 1 | | | 1 | | | | | | |
| | | | Myristyldimethylamine oxide | | | 0.3 | | | | | | | | |
| | | | Benzalkonium chloride(3) | | | | 1 | | | | | | | |
| | | | Polyglycerin fatty acid ester (4) | | | | | | 0.5 | | | | | |
| | | | Sucrose fatty acid ester | | | | | | | 1 | | | | |
| | | | Alkylpolyglycoside(6) | | | | | | | | 0.5 | | | |
| | | (C) | Succinic acid | 0.85 | 1 | 1.75 | 0.85 | 0.85 | 1 | 0.85 | 1 | | 0.85 | |
| | | (F) | Polyoxyethylenelaurylether sulfate(7) | | | | | 0.5 | | | | | | |
| | | | Polyoxyethylenelauryl ether(8) | | | | | | | | | | | 1 |
| | | | Water | Balance | Balance | Balance | Balance | Balance | Balance | Balance | Balance | Balance | Balance | Balance |
| | | | Total | 100 | 100 | 100 | 100 | 100 | 100 | 100 | 100 | 100 | 100 | 100 |
| Germicidal washing test | Test solution | | Available chlorine concentration (ppm) | 500 | 500 | 500 | 500 | 500 | 500 | 500 | 500 | 500 | 500 | 500 |
| | | | pH (20°C) | 6.5 | 6 | 5 | 6.5 | 6.5 | 6 | 6.5 | 6 | 11 | 6.5 | 11 |
| | Detergency | | Oil-stain washing rate (%) | 65 | 65 | 63 | 45 | 67 | 60 | 63 | 58 | 15 | 22 | 25 |
| | | | Protein-stain washing rate (%) | 60 | 62 | 59 | 40 | 62 | 63 | 61 | 62 | 12 | 21 | 20 |
| | Germicidal activity (Germicidal performance) | Minimum germicidal concentration (ppm) | *Bacillus subtilis* | 80 | 60 | 50 | 150 | 80 | 62.5 | 125 | 62.5 | 6000 | 1500 | 6000 |
| | | | Mold | 60 | 50 | 50 | 120 | 60 | 62.5 | 125 | 62.5 | 3000 | 1000 | 3000 |

(1): The available chlorine concentration is given in parentheses.

(2): Amphitol 20N (made by Kao Corporation effective amount: 35 %) was used to set the effective concentration to the value indicated in Table X1.

(3): Sanisol C (made by Kao Corporation effective amount: 50 %) was used to set the effective concentration to the value indicated in Table X1.

(4): MCA-750 (made by Sakamoto Yakuhin Kogyo Co., Ltd.) was used to set the effective concentration to the value indicated in Table X1.

(5): LWA1570 (made by Mitsubishi-Kagaku Foods Corporation) was used to set the effective concentration to the value indicated in Table X1.

(6): Mydol 12 (made by Kao Corporation effective amount: 40 %) was used to set the effective concentration to the value indicated in Table X1.

(7): Emal 20C (made by Kao Corporation effective amount: 25 %) was used to set the effective concentration to the value indicated in Table X1.

(8): Emulgen 106 (made by Kao Corporation) was used to set the effective concentration to the value indicated in Table X1.

Examples X9 to X12, Comparative Example X4

Of electrolytic oxidized water obtained by a diaphragm method, hypochlorous acid in water generated on the anode side (pH (25°C) 2.7, available chlorine concentration 50 ppm) was used, and adjusted to pH 11 by using 0.1 mol/L of aqueous solution of sodium hydroxide to obtain a composition represented by comparative example X4 of Table X2. Moreover, the above-mentioned hypochlorous acid in water was adjusted to pH 5 by using 1 mol/L of disodium succinate, and lauryldimethylamine oxide (the same composition as preferred embodiment X1) was added thereto so as to have a concentration of 25 ppm; thus, the composition of example X9 shown in Table X2 was obtained. In the same manner, the compositions of examples X10 to X12 were obtained. By using these, tests on germicidal property were carried out in the same manner as example X1; thus, when no growth of bacteria was observed, this case was evaluated as "◎" or "○", and when any growth was observed, this case was evaluated as "×". Table X2 shows the results of the evaluation.

Table X 2

| | | | Example | | | | Comparative example |
|---|---|---|---|---|---|---|---|
| | | | X9 | X10 | X11 | X12 | X4 |
| Blended components | (A) | Hypochlorous acid | 50ppm | 50ppm | 50ppm | 50ppm | 50ppm |
| | (B) | Lauryldimethylamine oxide* | 25ppm | | | | — |
| | | Polyglycerin fatty acid ester** | | 25ppm | | | |
| | | Sucrose fatty acid ester*** | | | 25ppm | | |
| | | Akylpolyglycoside**** | | | | 25ppm | |
| | (C) | Doisodium succinate | pH adjustable amount | pH adjustable amount | pH adjustable amount | pH adjustable amount | — |
| | | Sodium hydroxide | — | | | | pH adjustable amount |
| pH(20°C) | | | 5 | 5 | 5 | 5 | 11 |
| Germicidal activity (Germicidal performance) | *Bacillus subtilis* | | ◎ | ○ | ○ | ○ | × |
| | Mold | | ◎ | ○ | ○ | ○ | × |

*Amphitol 20N (made by Kao Corporation, effective amount 35 %)

**MCA-750 (made by Sakamoto Yakuhin Kogyo Co., Ltd.)

***LWA1570 (made by Mitsubishi-Kagaku Foods Corporation)

****Mydol 12 (made by Kao Corporation, effective amount 40 %)

Examples X13 to X20 and Comparative Examples X5 to X7

By using compositions made from components shown in Table X3, the following tests were carried out. Table X3 shows the results of the tests.

Each of the compositions was obtained by mixing the same amounts of a solution, which had been prepared by mixing predetermined amounts of an aqueous solution of sodium hypochlorite (available chlorine concentration 60000 ppm) and (B) component and/or (F) component, and then diluting this by adding ion exchanged water to two times the final blended concentration, and a solution of succinic acid, which had been prepared by diluting succinic acid with ion exchanged water to two times the final blended concentration.
Moreover, in each of the tests, sample solutions that had been diluted so as to set the compositions of Table X3 to the available chlorine concentrations of Table X3 were used. Here, the available chlorine concentration was measured based upon "Iodometry" in JIS K-0101. The respective components in Table X3 are the same as those listed in Table X1.

(XI) Detergency

Fat and oil (20 g), formed by mixing beef tallow and soybean oil at a volume ratio of 1:1, 0.25 g of mono-olein and 0.1 g of oil red were dissolved in 60 ml of chloroform to prepare an oil stain solution. A set of six sheets of clean slide glass (76 mm × 26 mm × 1 mm) was prepared and the mass of each of the sheets were measured to the unit of 1 mg. The sheets of slide glass were immersed into the oil stain solution at 25 ± 1°C sheet by sheet for approximately 2 seconds up to approximately 55 mm so that oil stain was allowed to adhere thereto, and the sheets of glass were then taken out. The excessive portion of the oil stain adhering to the lower portion of the sheet of slide glass was absorbed by clean cloth such as gauze and filter paper so that the adhesion of oil stain was maintained evenly; then, this was dried in air at 25 ± 1°C and the mass thereof was measured. These model stain glass plates were used in tests after they had been left and dried for a period from not less than one hour to not more than two hours. In this case, the amount of adhesion of oil stain per six sheets of model stain glass plates was set to 0.140 ± 0.010 g.

A sample aqueous solution (300 ml)(25°C) was poured into a 300 ml beaker, and a silicon hose to the top end of which an air stone was attached was dropped into the sample aqueous solution, and air was sent thereto by an air pump (flow rate: 1.5 liters/minute) to generate bubbles. The model stain glass plates were made in contact with overflowed bubbles for 5 minutes sheet by sheet, and this was rinsed by ion exchanged water at 25 ± 2°C for 30 seconds. The glass plates, which had been subjected to the rinsing process, was dried in air for one whole-day. The evaluation of detergency was carried out based upon the weights of the model stain glass plates before and after the washing process. In other words, the difference in weight before and after the washing process was found so that the detergency (%) was calculated based upon the following equation.

$$\text{Detergency (\%)} = (\text{Weight before washing} - \text{Weight after washing}) / \text{Amount of stain adhesion} \times 100$$

The detergency was found on the respective six sheets of slide glass, and the average value of the four sheets that remained after the maximum value and the minimum value had been excluded was determined as the detergency of the corresponding composition.

(X II) Germicidal property

*Bacillus subtilis* ATCC6633, which is spore-forming bacteria, was used, and a loop of bacteria, which had been preliminarily cultivated in an SCD agar (made by Nihonseiyaku Co., Ltd.) was suspended in 1 ml of sterilized water, and subjected to a heating treatment at 65°C for 30 minutes, and this was then washed twice while being subjected to centrifugal separation; thus, the resulting bacteria were used in the test ($10^5$ cell/ml). After 0.5 ml of this sample spore-containing solution was uniformly inoculated on one sheet of model stain glass plate that had been prepared in the same manner as the above-mentioned (X 1), and then dried in air to prepare a germicidal-test-use glass plate.

The germicidal-test-use glass plate was made in contact with bubbles of sample aqueous solution generated in the same manner as described in the above-mentioned (X I), and immediately after this process, was rinsed with sterilized water, a predetermined area (20 mm × 20 mm) of the glass plate was wiped with swab before the one side of glass face had been dried up, and this was dipped into 1 ml of sterilized water so that the accretion was suspended therein. Then, 25μl of each suspension was inoculated onto a micro-plate containing 0.2 ml of post-cultivation-use SCDLP medium (containing 3.3 % of sodium thiosulfate) (which is the same as that used in [R1]). This was cultivated at 30°C for 48 hours, and the growth of the bacteria was visually observed; thus, it was determined whether or not the bacteria had grown up on the micro-plate. When no growth of bacteria was observed, this case was evaluated as "◎ " or "○", and when any growth was observed, this case was evaluated as "×". Table X3 shows the results of the evaluation.

Table X 3

| | | | | Example | | | | | | | | Comparative example | | |
|---|---|---|---|---|---|---|---|---|---|---|---|---|---|---|
| | | | | X13 | X14 | X15 | X16 | X17 | X18 | X19 | X20 | X5 | X6 | X7 |
| Germicidal washing agent composition | Blended components(weight %) | (A) | Sodium hypochlorite[1] | 1.05 (1) | 1.05 (1) | 1.05 (1) | 1.05 (1) | 1.05 (1) | 1.05(1) | 1.05(1) | 1.05(1) | 1.05 (1) | 1.05 (1) | 1.05 (1) |
| | | (B) | Lauryldimethylamine oxide (2) | 2 | 2 | | | 2 | | | | 2 | | |
| | | | Myristyldimethylamine oxide | | | 1 | | | | | | | | |
| | | | Benzalkonium chlorite[3] | | | | 2 | | | | | | | |
| | | | Polyglycerin fatty acid ester(4) | | | | | | 2 | | | | | |
| | | | Sucrose fatty acid ester (5) | | | | | | | 1 | | | | |
| | | | Alkylpolyglycoside (6) | | | | | | | | 2 | | | |
| | | (C) | Succinic acid | 0.85 | 1 | 1.75 | 0.85 | 0.85 | 1 | 1 | 1 | | | |
| | | (F) | Polyoxyethylenelauryl ether sulfate[7] | | | | | 1 | | | | | 1 | |
| | | Polyoxyethylenelauryl ether[8] | | | | | | | | | | | | 1 |
| | | Water | | Balance | Balance | Balance | Balance | Balance | Balance | Balance | Balance | Balance | Balance | Balance |
| | | | Total | 100 | 100 | 100 | 100 | 100 | 100 | 100 | 100 | 100 | 100 | 100 |
| Germicidal washing test | Test Solution | | Available chlorine concentration (ppm) | 200 | 200 | 200 | 200 | 200 | 200 | 200 | 200 | 200 | 200 | 200 |
| | | | pH (20°C) | 6.5 | 6 | 5 | 6.5 | 6.5 | 6 | 6 | 6 | 11 | 11 | 11 |
| | Detergency | | Detergency (%) | 58 | 58 | 56 | 43 | 60 | 57 | 58 | 55 | 40 | 26 | 22 |
| | Germicidal activity (germicidal performance) | | *Bacillus subtilis* | ◎ | ◎ | ◎ | ◎ | ◎ | ○ | ○ | ○ | × | × | × |

In the table, (1) to (8) are the same as those in table X1.

In the Table, (1) to (8) are the same as those in Table X1.

Examples X21 to X24

Moreover, of so-called electrolytic oxidized water obtained by a diaphragm method, hypochlorous acid in water generated on the anode side (pH (25°C) 2.7, available chlorine concentration 50 ppm) was used, and adjusted to pH 5 by using 1 mol/L of aqueous solution of disodium succinate, and to this was added (B) component so as to set the concentration thereof to 200 ppm; thus, compositions of examples X21 to X24 of Table X4 were obtained. By using these, tests on germicidal property were carried out in the same manner as examples X13 to X20. Table X4 shows the results of the tests.

Here, in the present examples, any of the tests were carried out by setting the contact time between bubbles and the germicidal-test-use glass plate to 10 minutes with the temperature of the bubble-forming-use sample solution being set to 50°C.

Table X 4

| | | | Example | | | |
|---|---|---|---|---|---|---|
| | | | X21 | X22 | X23 | X24 |
| Blended components | (A) | Hypochlorous acid | 50ppm | 50ppm | 50ppm | 50ppm |
| | (B) | Lauryldimethylamine oxide* | 200ppm | | | |
| | | Polyglycerin fatty acid ester** | | 200ppm | | |
| | | Sucrose fatty acid ester*** | | | 200ppm | |
| | | Alkylpolyglycoside**** | | | | 200ppm |
| | (C) | Disodium succinate | pH adjustable amount | pH adjustable amount | pH adjustable amount | pH adjustable amount |
| pH(20°C) | | | 5 | 5 | 5 | 5 |
| Germicidal activity(Germicidal performance) | | *Baccillus subtilis* | ◎ | ○ | ○ | ○ |

*Amphitol 20N (made by Kao Corporation, effective amount 35 %)

**MCA-750 (made by Sakamoto Yakuhin Kogyo Co., Ltd.)

***LWA1570 (made by Mitsubishi-Kagaku Foods Corporation)

****Mydol 12 (made by Kao Corporation, effective amount 40 %)

## Claims

1. A method for sterilizing microbes, comprising bringing an aqueous solution comprising (A) hypochlorous acid and/or a salt thereof, (B) a surfactant and (C) a pH adjusting agent in contact with microbes.

2. The method according to claim 1, wherein said aqueous solution has a pH value (25°C) of 3 to 8.

3. The method according to claim 1 or 2, wherein said aqueous solution has an available chlorine concentration of 5 to 5000 ppm.

4. The method according to any one of claims 1 to 3, wherein said pH adjusting agent(c) is an organic acid or a salt thereof.

5. The method according to claim 4, wherein said organic acid or a salt thereof is a saturated dibasic acid or a salt thereof.

6. The method according to any one of claims 1 to 5, wherein said surfactant (B) is at least one selected from the group consisting of an amphoteric surfactant, a cationic surfactant and a nonionic surfactant.

7. The method according to any one of claims 1 to 5, wherein said surfactant (B) is an amine oxide.

8. The method according to any one of claims 1 to 5, wherein said surfactant (B) is a polyhydric alcohol derivative surfactant.

9. The method according to any one of claims 1 to 8, which is used for carrying out a germicidal process on hard surfaces.

10. The method according to any one of claims 1 to 8, which is used for carrying out a mold-eliminating process.

11. The method according to any one of claims 1 to 8, which is used for carrying out a germicidal process on an automatic washing device.

12. The method according to any one of claims 1 to 8, which is used for carrying out a germicidal process on perishable foodstuff.

13. The method according to any one of claims 1 to 8, which is used for carrying out a germicidal process on textiles and fabrics.

14. The method according to any one of claims 1 to 8, which is used for carrying out a germicidal process on medical equipment.

## INTERNATIONAL SEARCH REPORT

International application No.
PCT/JP00/08717

A. CLASSIFICATION OF SUBJECT MATTER
Int.Cl[7] A01N59/08, A01N25/30, A01N25/02, A01N33/24, A61L2/18, A23L3/358, D06M11/11

According to International Patent Classification (IPC) or to both national classification and IPC

B. FIELDS SEARCHED

Minimum documentation searched (classification system followed by classification symbols)
Int.Cl[7] A01N59/08, A01N25/30, A01N25/02, A01N33/24, A61L2/18, A23L3/358, D06M11/11

Documentation searched other than minimum documentation to the extent that such documents are included in the fields searched

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP, 38-6268, B1 (Sanyo Yushi Kogyo K.K.), 17 May, 1963 (17.05.63), page 2, left column, line 17 to page 3 (Family: none) | 1,3,9-14 |
| X | JP, 52-78905, A (Lion Corp.), 02 July, 1977 (02.07.77), page 1, left column, line 1 to page 2, upper right column, line 1 (Family: none) | 1,3,9-14 |
| X | US, 4271030, A (UNILEVER N.V.), 02 June, 1981 (02.06.81), Column 2, line 17 to Column 3, line 29 & DE, 2849225, A & NL, 7811311, A & BR, 7807568, A & FI, 7803502, A & GB, 2015603, A & IT, 1160940, B & JP, 54-88889, A | 1,3,6,7,9-14 |
| X<br>Y | WO, 87/5187, A1 (AUCHINCLOSS T.R.), 11 September, 1987 (11.09.87), page 3, line 5 to page 6, line 17 & EP, 260293, A1 & CA, 1290243, C | 1-6,8-14<br>7 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 27 February, 2001 (27.02.01) | 06 March, 2001 (06.03.01) |
| Name and mailing address of the ISA/ Japanese Patent Office | Authorized officer |
| Facsimile No. | Telephone No. |

Form PCT/ISA/210 (second sheet) (July 1992)

## INTERNATIONAL SEARCH REPORT

International application No.
PCT/JP00/08717

C (Continuation). DOCUMENTS CONSIDERED TO BE RELEVANT

| Category* | Citation of document, with indication, where appropriate, of the relevant passages | Relevant to claim No. |
|---|---|---|
| | & KR, 9200863, B1 & GB, 2187098, A<br>& JP, 63-502662, A & US, 4822512, A | |
| Y | JP, 2-111708, A (Jibukomu K.K.),<br>24 April, 1990 (24.04.90),<br>page 1, left column, line 1 to right column, line 10; page 2, lower right column, lines 5 to 19 (Family: none) | 1-14 |
| Y | JP, 4-360672, A (Juichiro YAGI),<br>14 December, 1992 (14.12.92),<br>page 1, Column 1, line 1 to Column 2, line 46<br>(Family: none) | 1-14 |
| Y | JP, 8-164189, A (Morinaga Milk Ind. Co., Ltd.),<br>25 June, 1996 (25.06.96),<br>page 2, Column 1, lines 1 to 22 (Family: none) | 1-14 |
| X<br>Y | JP, 9-154922, A (Tomey Technology Corporation),<br>17 June, 1997 (17.06.97),<br>page 3, Column 4, lines 12 to 40; page 4, Column 6, lines 24 to 45 (Family: none) | 1-6,9-14<br>7,8 |
| Y | JP, 11-49619, A (TOSOH CORPORATION),<br>23 February, 1999 (23.02.99),<br>page 2, Column 2, lines 14 to 26 (Family: none) | 8 |
| X<br>Y | JP, 11-148097, A (Kao Corporation),<br>02 June, 1999 (02.06.99),<br>page 2, Column 1, line 1 to Column 2, line 50; page 3, Column 4, lines 19 to 22 (Family: none) | 1-6,8-14<br>7 |
| X<br>Y | JP, 11-148098, A (Kao Corporation),<br>02 June, 1999 (02.06.99),<br>page 2, Column 1, line 1 to page 4, Column 5, line 39,<br>(Family: none) | 1-4,6-14<br>5 |
| Y | JP, 11-188083, A (Yoshiya OKAZAKI),<br>13 July, 1999 (13.07.99),<br>page 2, Column 1, lines 1 to 10 (Family: none) | 1-14 |
| Y | JP, 11-228316, A (Clean Chemical K.K.),<br>24 August, 1999 (24.08.99),<br>page 2, Column 1, lines 1 to 42 (Family: none) | 1-14 |

Form PCT/ISA/210 (continuation of second sheet) (July 1992)